# EUROPEAN PATENT APPLICATION

(11) **EP 2 113 208 A2**
(43) Date of publication of application: **04.11.2009**
(21) Application number: 09006037.7
(22) Date of filing: 30.04.2009
(51) Int. Cl.: A61B 17/122, A61B 17/128

(54) **Multiple clip device and multiple clip application apparatus**

(30) Priority: 02.05.2008 JP 2008120565; 07.07.2008 JP 2008177160; 11.09.2008 JP 2008233656
(71) Applicant: Fujifilm Corporation, Tokyo 106-0031 (JP)
(72) Inventor: Cui, Shengfu, Saitama-shi Saitama (JP); Ida, Takayuki, Saitama-shi Saitama (JP); Kunuki, Yoshiyuki, Saitama-shi Saitama (JP)
(74) Representative: Klunker . Schmitt-Nilson . Hirsch

(57) **Abstract**

A multiple clip device for hemostatic clamping of a lesion of a patient's body includes plural clips (19) fastened to one another in a train, having first and second clips, a proximal end portion (26) of the first clip (19A) being engaged with two claws (23) of a distal end portion of the second clip (19B-19E). A dummy clip (47, 131) has claws (47b, 131b) in a shape of the claws of the clips, and is engaged with a proximal end portion of a final clip among the clips by the claws thereof. An end connector (52, 134) is for retention of the dummy clip, and for fastening in a removable manner to a shaft head (57, 123, 136) at an end of an operating wire (12) inserted in a flexible sheath (11) of a multiple clip application apparatus. Specifically, the multiple clip application apparatus is constituted by the operating wire and the multiple clip device fastened thereto.

## Description

### BACKGROUND OF THE INVENTION

### 1. Field of the Invention

The present invention relates to a multiple clip device and a multiple clip application apparatus. More particularly, the present invention relates to a multiple clip device which includes plural clips, in which one of the clips is applied to a lesion in a human body in a successive manner, and which can be managed for maintenance periodically or for check of performance, and a multiple clip application apparatus.

### 2. Description Related to the Prior Art

Tissue clamping is known as medical treatment of a lesion in a gastrointestinal tract by use of an endoscope. In the clamping, a clip of a small size is used to clip the lesion for the purpose of hemostasis, suture and the like. U.S.P. No. 6,814,742 (corresponding to JP-A 2002-191609 and JP-A 2007-222649) and U.S.P. Pub. No. 2005/033312 (corresponding to JP-A 2005-021587) disclose a hemostatic clip application apparatus, which includes a flexible sheath, an operating wire and a handle assembly. The flexible sheath is loaded with the clip. The operating wire is fastened to a proximal end of the clip in the flexible sheath. The handle assembly is used to operate the flexible sheath and the operating wire.

A pair of arms in the clip are formed by bending a strip of metal plate for opening and closing. When in a natural state, the arms are open according to their resiliency. Claws are disposed at ends of the arms for mesh with one another when closed. A turn, which extends between the arms in the metal plate, is deformed resiliently. The clip is loaded in the flexible sheath in a closed state of the arms. A fastening mechanism is used to fasten the clip to the operating wire.

A forceps channel is formed through the endoscope. The flexible sheath loaded with the clip is entered in a gastrointestinal tract of a patient's body through the forceps channel of the endoscope. The clip is advanced through the sheath end of the flexible sheath by moving the operating wire, to open the arms with resiliency of a turn formed between the arms. When the operating wire is pulled with pressure of the claws to the lesion, the clip is closed by pushing the arms with the sheath end. The claws at the end of the arms clip the lesion. Subsequently, the operating wire is pulled to break the fastening mechanism to disconnect the operating wire from the clip.

Only one clip is loaded in the hemostatic clip application apparatus of a conventional structure. If the clamping for hemostasis of plural steps in a successive manner is required, the flexible sheath is removed from the forceps channel of the endoscope after each step of the clamping. A new clip must be fastened to the wire end of the operating wire and loaded in the flexible sheath to insert the flexible sheath through the forceps channel. This is a very complicated process.

A multiple hemostatic clip application apparatus is suggested for the clamping without reloading operation of the clip for the purpose of raising efficiency of stepwise preparation for the clamping. JP-A 2006-187391 discloses the multiple hemostatic clip application apparatus in which plural clips are loaded in the flexible sheath, and advanced consequently to the outside of the flexible sheath for the clamping.

Among the clips in JP-A 2006-187391, a second clip is fastened to a first clip by engagement of a claw of the second clip in a coupling hole in the first clip. A final clip is connected with the operating wire. The first clip is advanced from out of the flexible sheath by movement of the operating wire. When the operating wire is pulled, the first clip clips the lesion in a manner similar to the hemostatic clip application apparatus. After clamping of the first clip, the second clip is advanced out of the flexible sheath. The arms of the second clip are shifted to open for separation of the first clip.

To fasten the second clip to the operating wire, the fastening mechanism is disposed at the wire end. The fastening mechanism includes the arms of metal for opening and closing in a manner similar to the clips. The claws at the ends of the arms are engaged with coupling holes of the final clip. Fastening between the final clip and the operating wire is the same as that between the clips. This structure is effective in higher working efficiency than the use of the fastening mechanism of U. S. P. No. 6,814,742 (corresponding to JP-A 2002-191609 and JP-A 2007-222649) and U.S.P. Pub. No. 2005/033312 (corresponding to JP-A 2005-021587), because the operation of release is common between the clips including the first clip and the final clip.

There is a problem in the multiple hemostatic clip application apparatus of JP-A 2006-187391. The fastening mechanism, connected between the final clip and the operating wire, has low durability because of a strip of a metal plate. When the fastening mechanism deteriorates, failure in fastening between the clips and the operating wire occurs so that the clips will not move even in pulling the operating wire. Furthermore, the operating wire must be exchanged when the fastening mechanism becomes unusable, because the fastening mechanism is positioned at the wire end. The suitability of the multiple hemostatic clip application apparatus for maintenance will be low. The cost of the multiple hemostatic clip application apparatus will be considerably high due to the exchange of the operating wire.

### SUMMARY OF THE INVENTION

In view of the foregoing problems, an object of the present invention is to provide a multiple clip device which includes plural clips, in which one of the clips is applied to a lesion in a human body in a successive manner, and which can be managed for maintenance periodically or for check of performance, and a multiple clip application apparatus.

In order to achieve the above and other objects and advantages of this invention, a multiple clip device includes plural clips fastened to one another in a train, having first and second clips, a proximal end portion of the first clip being engaged with two claws of a distal end portion of the second clip. A dummy clip has claws in a shape of the claws of the clips, and is engaged with a proximal end portion of a final clip among the clips by the claws thereof. There is an end connector for retention of the dummy clip, and for fastening in a removable manner to a shaft head at an end of an operating wire inserted in a flexible sheath of a clip application apparatus.

The end connector has a proximal end portion inclined with a decreasing diameter in a direction of insertion into the flexible sheath.

The shaft head includes a first shaft head portion with a decreasing width at a distal end portion, and the first shaft head portion is fitted in the end connector for connection with the operating wire.

The shaft head includes a second shaft head portion, positioned behind the first shaft head portion, for contacting a proximal end of the end connector. The end connector includes a clamping wall engaged with a proximal end of the first shaft head portion by clamping the operating wire between the first and second shaft head portions.

An interval between the first and second shaft head portions is smaller than a length of the clamping wall in an axial direction.

Also, a multiple clip application apparatus is provided, and includes an operating wire, inserted in a flexible sheath, and having a shaft head at an end thereof. A multiple clip device is connected with the operating wire, and loaded in the flexible sheath. The multiple clip device includes plural clips fastened to one another in a train, having first and second clips, a proximal end portion of the first clip being engaged with two claws of a distal end portion of the second clip. A dummy clip has claws in a shape of the claws of the clips, and is engaged with a proximal end portion of a final clip among the clips by the claws thereof. There is an end connector for retention of the dummy clip, and for fastening in a removable manner to the shaft head.

The flexible sheath is substantially cylindrical. The second shaft head portion is substantially cylindrical, and has a diameter greater than a length of a diagonal of the proximal end of the first shaft head portion and substantially equal to an inner diameter of the flexible sheath, and the first shaft head portion is engaged with the end connector by pressing the second shaft head portion.

The second shaft head portion includes an inclined surface, disposed at an end thereof on a proximal side, having a gradually decreasing diameter toward the proximal side in the operating wire.

Furthermore, plating is provided on a distal end portion of the operating wire including the first and second shaft head portions.

In one preferred embodiment, a multiple clip application apparatus includes an operating wire, inserted in a cylindrical flexible sheath, and having a first shaft head portion with a decreasing width. A multiple clip device has plural clips fastened to one another in a train, and an end connector disposed on a final one of the clips disposed finally, wherein the first shaft head portion is fitted in the end connector for connection with the operating wire and loading in the flexible sheath.

Also, a dummy clip is provided, and includes a support fastened to a proximal end of a clip contained in a housing on a proximal side. There is a connector for receiving insertion of a shaft head at a distal end of an operating wire for operating the clip, and for connection with the shaft head by resilient deformation.

Also, a multiple clip holder includes a multiple clip device, having plural clips engaged with one another in a train, plural retaining rings for keeping the clips fastened by covering engaged portions of the clips, and the dummy clip fastened to a final one of the clips disposed finally. A housing has a through bore, having an inner diameter slightly greater than an outer diameter of the retaining rings, for containing the multiple clip device, and a dummy clip cavity for containing the connector to receive access of the shaft head.

Also, a clip loading method of loading the multiple clip device in the flexible sheath from the multiple clip holder is provided. In the clip loading method, the shaft head portion is fastened to the dummy clip by resiliently deforming the connector upon insertion in the housing with pressure of the shaft head portion to the connector. The multiple clip device is loaded in the flexible sheath from the housing by moving the flexible sheath and the housing toward a distal end side relative to the operating wire after setting the flexible sheath in the receiving sleeve of the housing.

Consequently, the multiple clip device of the invention can be managed for maintenance periodically or for check of performance, because of the use of the dummy clip disposed between the plural clips and the operating wire.

### BRIEF DESCRIPTION OF THE DRAWINGS

The above objects and advantages of the present invention will become more apparent from the following detailed description when read in connection with the accompanying drawings, in which:
Fig. 1 is a perspective view illustrating a multiple hemostatic clip application apparatus;
Fig. 2A is a vertical section illustrating the multiple hemostatic clip application apparatus on a side of a sheath end;
Fig. 2B is a vertical section illustrating the same as Fig. 2A but as viewed with a difference of a right angle;
Fig. 3A is a vertical section illustrating the multiple hemostatic clip application apparatus on the side of the sheath end in enlargement;
Fig. 3B is a vertical section illustrating the same as Fig. 3A but as viewed with a difference of a right angle;
Fig. 4 is a perspective view illustrating a clip and a retaining ring;
Fig. 5A is a front elevation illustrating the retaining ring;
Fig. 5B is a vertical section illustrating the retaining ring;
Fig. 5C is a cross section illustrating the retaining ring;
Fig. 5D is a top plan illustrating the retaining ring;
Fig. 6 is a perspective view, partially broken, illustrating a final hemostatic clip mechanism, a fastening clip mechanism and an operating wire;
Fig. 7 is a perspective view illustrating the fastening clip mechanism;
Fig. 8A is a vertical section illustrating a handle assembly;
Fig. 8B is a vertical section illustrating the same as Fig. 8A but as viewed with a difference of a right angle;
Fig. 9A is a vertical section illustrating a first step in a sequence of clamping with the multiple hemostatic clip application apparatus;
Fig. 9B is a vertical section illustrating a step of opening the clip mechanism in the clamping sequence;
Fig. 9C is a vertical section illustrating clamping of a lesion in the clamping sequence;
Fig. 9D is a vertical section illustrating a step of separating the clip mechanism from second and other clip mechanisms;
Fig. 10 is a perspective view illustrating a multiple clip holder;
Fig. 11 is a vertical section illustrating the multiple clip holder;
Fig. 12 is a perspective view illustrating the multiple clip holder;
Fig. 13 is an exploded perspective view illustrating a multiple clip device, a housing and a pull rod structure;
Fig. 14A is a vertical section illustrating entry of the multiple clip device into a coupling device from the housing;
Fig. 14B is a vertical section illustrating removal of the housing from the coupling device;
Fig. 15A is a vertical section illustrating insertion of a flexible sheath in the coupling device;
Fig. 15B is a vertical section illustrating fastening of the multiple clip device to the operating wire;
Fig. 16A is a vertical section illustrating insertion of the flexible sheath into the coupling device;
Fig. 16B is a vertical section illustrating completion of the entry of the multiple clip device into the flexible sheath from the coupling device;
Fig. 17 is a perspective view illustrating a second preferred embodiment with a final one of the clip mechanisms, the fastening clip mechanism, and the operating wire;
Fig. 18 is a plan illustrating a support for fastening;
Fig. 19 is a plan illustrating a wire end of the operating wire;
Fig. 20 is a cross section illustrating the coupling device for connecting a shaft head to the support for fastening;
Fig. 21 is a vertical section illustrating entry of the multiple clip device from the coupling device into the flexible sheath;
Fig. 22 is a perspective view illustrating the fastening clip mechanism according to a third preferred embodiment;
Fig. 23 is a perspective view illustrating the multiple clip holder;
Fig. 24A is a vertical section illustrating a first step in a sequence of loading the flexible sheath with the multiple clip device from the multiple clip holder;
Fig. 24B is a vertical section illustrating an intermediate step in the loading sequence of the multiple clip device;
Fig. 24C is a vertical section illustrating a final step in the loading sequence of the multiple clip device;
Fig. 25 is a vertical section illustrating the multiple clip device in a housing;
Fig. 26 is a vertical section illustrating loading of the flexible sheath with the multiple clip device.

### DETAILED DESCRIPTION OF THE PREFERRED EMBODIMENT(S) OF THE PRESENT INVENTION

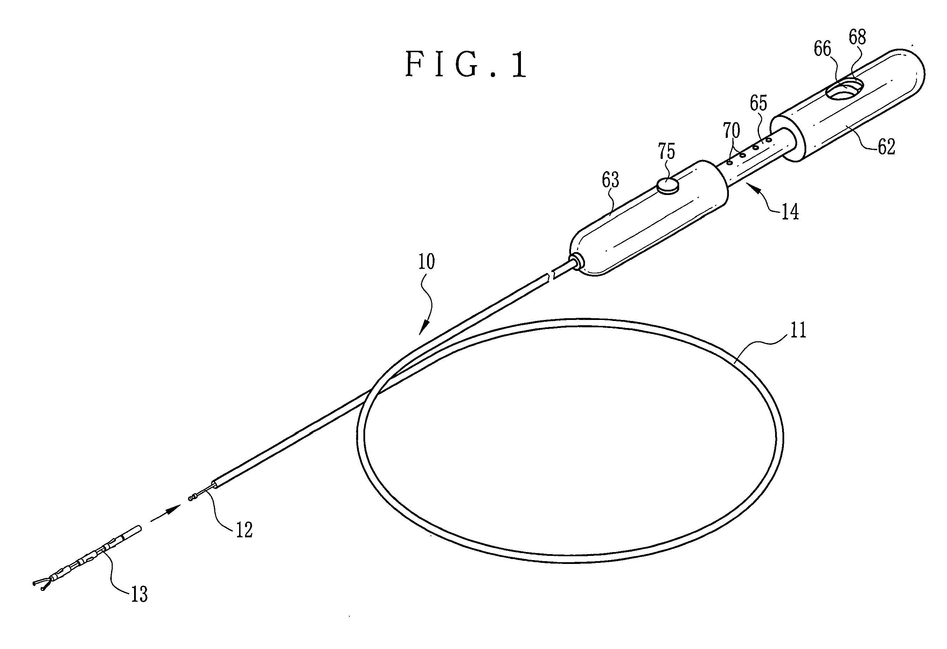

A multiple hemostatic clip application apparatus 10 of the invention is described first. In Fig. 1, the multiple hemostatic clip application apparatus 10 includes a cylindrical flexible sheath 11, an operating wire 12, a multiple clip device 13 or assembled clip device or clip train, and a handle assembly 14. The operating wire 12 is inserted through the flexible sheath 11. The multiple clip device 13 is loaded in the flexible sheath 11 and fastened to the operating wire 12. The handle assembly 14 is manually operable for pulling the flexible sheath 11 and the operating wire 12.

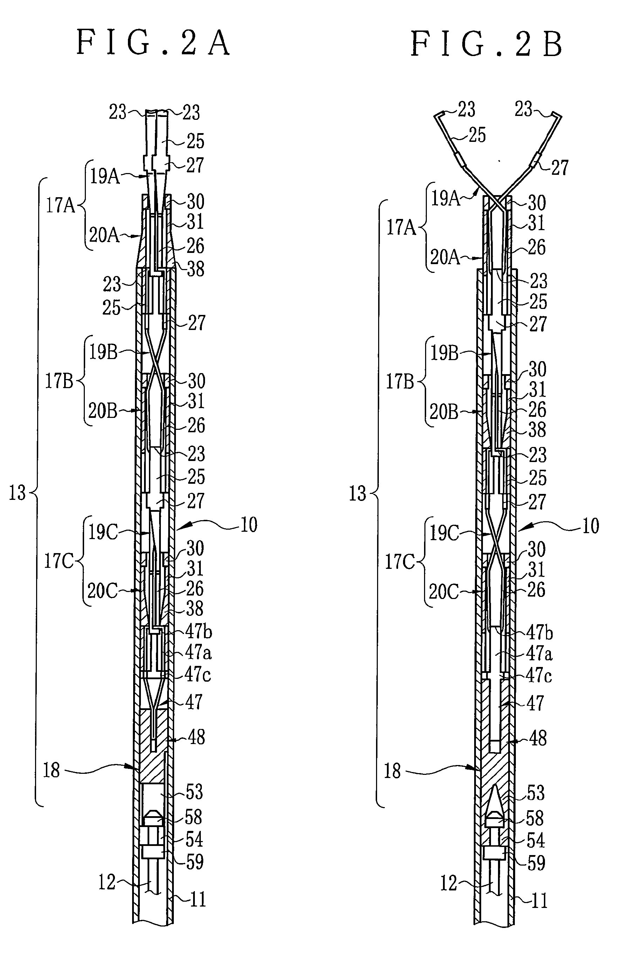

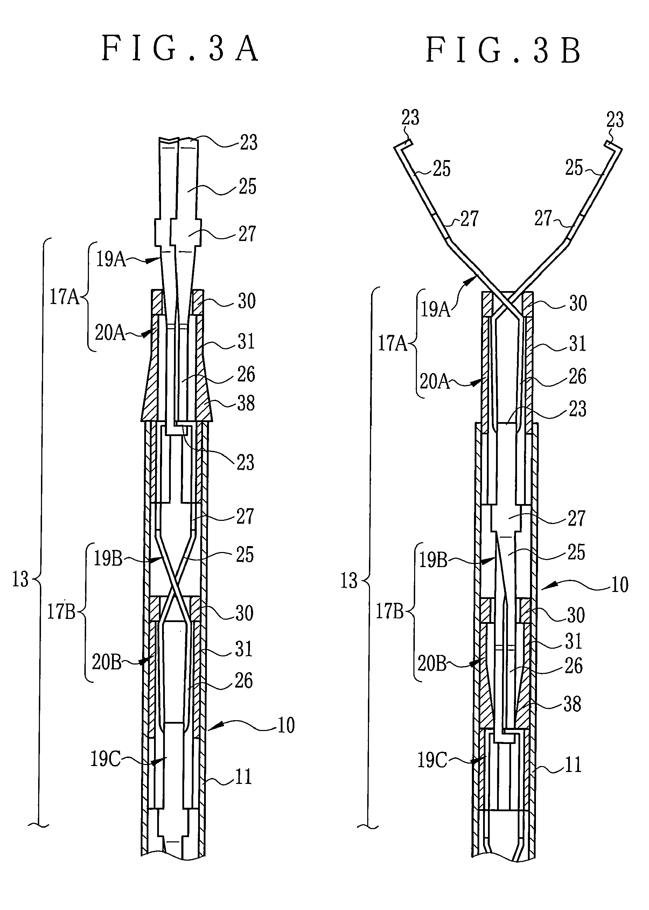

In Figs. 2A, 2B, 3A and 3B, a sheath end of the flexible sheath 11 loaded with the multiple clip device 13 is illustrated. A clip 19A protrudes from the flexible sheath 11, and stands by for hemostatic clamping in Fig. 2A. In Fig. 2B, the sheath end is viewed at an angle with a difference of 90 degrees from Fig. 2A. In Figs. 3A and 3B, the sheath end of Figs. 2A and 2B is illustrated in enlargement.

The multiple clip device 13 includes a train of hemostatic clip mechanisms 17 and a fastening clip mechanism 18. The clip mechanisms 17 are hemostatic clip mechanisms 17A, 17B and 17C fastened to one another in the train. The fastening clip mechanism 18 is fastened to the clip mechanism 17C disposed at the proximal end. The clip mechanisms 17 include clips 19 and retaining rings 20. The clips 19 are the clip 19A and clips 19B and 19C. The retaining rings 20 are retaining rings 20A, 20B and 20C, and set on the outside of the clips 19. The clip mechanisms 17A-17C are fastened to one another by engagement of a first one with a second one of the clips 19. The fastening clip mechanism 18 is fastened to the clip 19C disposed finally. The retaining rings 20A-20C maintain the fastened state by covering engaged portions of the clips 19A-19C.

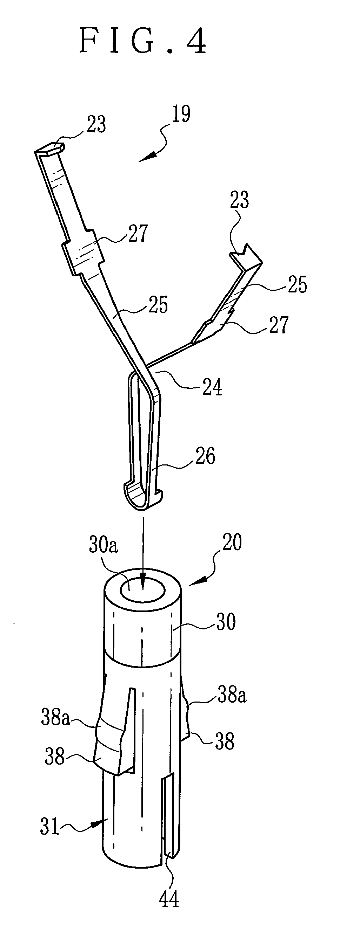

In Fig. 4, each of the clips 19 includes claws 23 and a turn 26 which extends in a twisted form with an angle of 180 degrees from the claws 23. At the turn 26, the clip 19 is closed. At first, a strip of a single plate is bent at an angle of 180 degrees, to form a turn as a closed portion. Segment portions extending at ends of the plate are intersected with one another, and opposed to one another by curving, to define the claws 23. In the clip 19, a crossed portion 24 is formed by crossing the segment portions. Arms 25 in the clip 19 extend at the free ends. Side projections 27 are formed with middle portions of the arms 25 to define a greater arm width. An example of material for the clip 19 is metal with biocompatibility, for example SUS 631 as stainless steel for springs.

An arm shifting sleeve 30 of the retaining rings 20, which is disposed around the crossed portion 24 of the clip 19, pushes the arms 25 and moves toward the claws 23 at a predetermined length. The arms 25 and the claws 23 become closed for ensured clamping at the claws 23.

The claws 23 are a projection and a notch in the combination for the purpose of capturing an object. In Figs. 3A and 3B, the arms 25 of the clip 19 are formed with an increasing width from the crossed portion 24 toward the side projections 27.

The side projections 27 have a greater width than a portion of both of first and second openings of each of the retaining rings 20 formed axially in contact with the side projections 27. A portion of the clip 19 except for the side projections 27 can enter the retaining ring 20. In contrast, the side projections 27 cannot enter the retaining ring 20 in any one of the two directions.

In Figs. 2A, 2B, 3A and 3B, the clip 19B is fastened to the clip 19A by engagement of the claws 23 of the clip 19B on the turn 26 of the clip 19A and retention of the claws 23 on the retaining ring 20A. The claws 23 of the clip 19B are engaged with each other transversely to the turn 26 of the clip 19A. After fastening, the clip 19B is oriented with a difference of a right angle from the clip 19A. Similarly, the clip 19C of fastening is oriented with a difference of a right angle from the clip 19B.

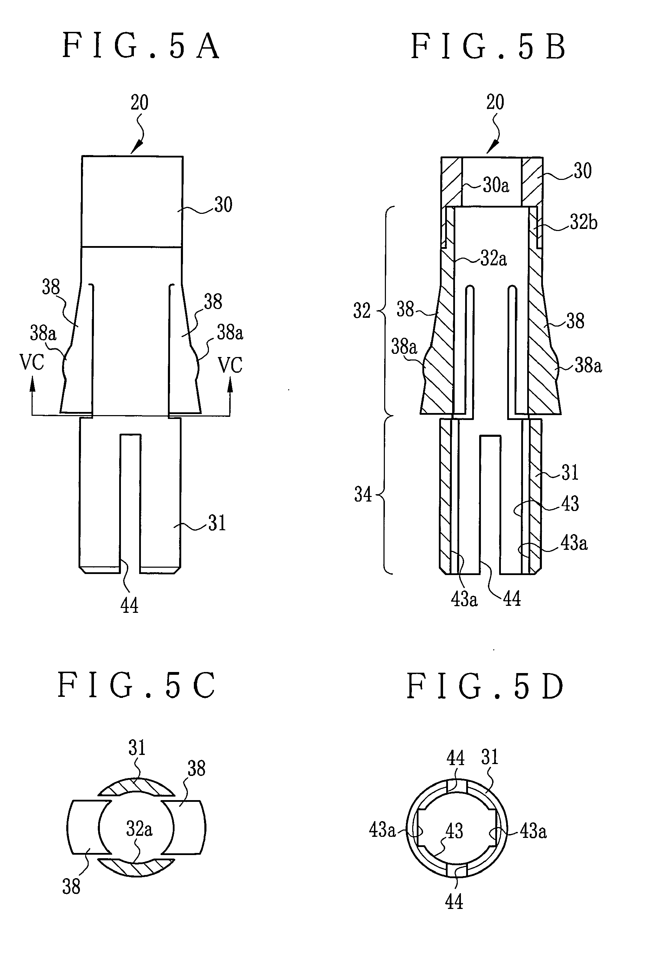

The retaining ring 20 is inserted in the flexible sheath 11 movably back and forth, and maintains the fastened state between two of the clips 19 by covering their engaged portion. The retaining ring 20 has an outer diameter substantially equal to an inner diameter of the flexible sheath 11, and can move back and forth smoothly in the flexible sheath 11 when the clips 19 move. In Figs. 4 and 5A-5D, the retaining ring 20 is illustrated. Fig. 5C is a section taken on line VC-VC in Fig. 5A.

An end connector 31 is combined with the arm shifting sleeve 30 to constitute the retaining ring 20. The arm shifting sleeve 30 as a part of metal is fixed firmly on the end connector 31 as a part of resin. The end connector 31 operates for setting the clips 19 in the retaining ring 20 and maintaining the connected state. The arm shifting sleeve 30 operates to close the clips 19 for clamping. Note that the retaining ring 20 may be a single piece which can operate for both of the arm shifting sleeve 30 and the end connector 31.

The arm shifting sleeve 30 is formed from metal and secured to the end of the retaining ring 20. A small bore 30a is defined in the arm shifting sleeve 30, greater than a width of the clip 19 near to the crossed portion 24, and smaller than the width of the side projections 27. Thus, the arm shifting sleeve 30 can move near to the crossed portion 24 of the clip 19, but cannot moved up to the distal end beyond the side projections 27. In short, the side projections 27 operate as a stopper for predetermining a limit of the retaining ring 20 in movement relative to the clip 19.

The arm shifting sleeve 30 is positioned suitably near to the crossed portion 24 of the clip 19. The arm shifting sleeve 30 moves from its initial position toward the side projections 27 beyond the crossed portion 24 with an increasing interval of the arms 25 of the clip 19, and shifts the arms 25 of the clip 19 for closing and clamping. An example of material for the arm shifting sleeve 30 is metal with biocompatibility, for example stainless steel SUS 304. Force of friction can be exerted by use of the metal for the arm shifting sleeve 30 in view of shifting the clip 19.

The end connector 31 is a cylindrically molded part of resin. The end connector 31 includes a first region 32 and a second region 34. The first region 32 supports a first one of the clips 19. The second region 34 supports a second one of the clips 19 fastened to the first.

A large bore 32a is defined within the first region 32 at a size greater than a bore in the arm shifting sleeve 30 and sufficient for containing the turn 26 of the clips 19. A stepped end 32b is formed with an outer surface of the first region 32 for fitting the arm shifting sleeve 30. The end connector 31 is fitted on the arm shifting sleeve 30 with such tightness that those do not disengaged in a state loaded in the flexible sheath 11 and during the clamping.

Fins 38 or skirt portions are disposed in the first region 32 and deploy with an inclination from an axis of the retaining ring 20. A proximal fin end of the fins 38, which is located in an upper position in Figs. 5A and 5B, is extended from a main portion of the end connector 31. A distal fin end is partially separate from the main portion, and shiftable radially for deployment and stowage as illustrated in Fig. 5C. Two of the fins 38 are arranged about the axis of the end connector 31 symmetrically. Positions of the fins 38 according to the axial direction of the clips 19 are equal, namely as viewed in the vertical direction on the drawing sheet of Fig. 5A.

In Figs. 5A and 5B, the fins 38 are in a deployed position when left to stand naturally without application of external force. The inside of the first region 32 of the end connector 31 is a cylindrical space as illustrated in Fig. 5B. In movement of the retaining rings 20 into the flexible sheath 11, the fins 38 are depressed and stowed inside in the state of the retaining ring 20B illustrated in Fig. 3B. Inner surfaces of the fins 38 depress lateral edges of the turn 26 of the clip 19B retained in the first region 32, so as to maintain the clip 19B in an immovable manner with respect to a rotational direction and axial direction in the retaining ring 20B. Note that it is possible to position the fins 38 in tight contact with a rear one of the clips retained in the second region 34.

In Fig. 3A, the retaining ring 20A is illustrated as the first of the retaining rings 20. The fins 38, upon advance out of the sheath end of the flexible sheath 11, become deployed by their resiliency. The fins 38 release the clip 19A from retention, and come to have a greater width than the inner diameter of the flexible sheath 11, to prevent the retaining ring 20A from returning into the flexible sheath 11. Then the operating wire 12 is pulled to move back the clip 19A. The retaining ring 20A moves forwards relative to the clip 19A, and shifts the clip 19A for closing and clamping.

Thus, the fins 38 should have such resiliency that those are stowed inwards when located in the flexible sheath 11, and become deployed when shifted out of the flexible sheath 11 and released from pressure. Also, the fins 38 must have such rigidity as to retain the clips 19 in the flexible sheath 11 and stand with resistance against reaction of the clamping force of the clips 19 at the sheath end.

In view of this aspect, the end connector 31 is formed from a material which has biocompatibility and has sufficient resiliency and rigidity for the performance of the fins 38. Also, the shape of the end connector 31 is predetermined to have sufficient resiliency and rigidity for the performance of the fins 38. A preferable example of the material for the end connector 31 is polyphenylsulfone (PPSU or PPS). It is preferable to form the end connector 31 by molding as one piece for facilitating the manufacture.

Projections 38a are formed with outer surfaces of the fins 38 and protrude radially. The projections 38a operate to close the fins 38 in the course of loading the flexible sheath 11 with the retaining ring 20 through the sheath end to prevent interference of a distal end of the fins 38 on the sheath end. Also, the projections 38a reduce friction of the retaining ring 20 with the flexible sheath 11 by contacting its inner surface.

The second region 34 is positioned on the proximal side from the first region 32. The second region 34 maintains the engaged state of the turn 26 of the first of the clips 19 with the claws 23 of the second of the clips 19 succeeding to the first retained in the first region 32.

A size of the second region 34 is predetermined substantially equal to a length of movement in which the arm shifting sleeve 30 set in the initial position relative to the clip 19 must move to complete clamping of the clip 19 by closing. The second region 34 of the retaining ring 20 maintains fastening of two of the clips 19 to transmit pulling force of a second one of the clips 19 forwards to a first one of the clips 19 while the clips 19 are moved back relative to the retaining ring 20 for clamping. When the clamping is completed, the engaged portion of the clips 19 is released from the second region 34, to disengage the first from the second of the clips 19.

In Fig. 5D, a center hole 43 is formed in the second region 34 and has a bore substantially equal to that of an end of the first region 32 on the proximal side. Two inner grooves 43a are formed in the second region 34 and opposed to one another. The inner grooves 43a contain the arms 25 of the clip 19 retained in the second region 34 with the claws 23 in the closed position.

The inner grooves 43a are located in the direction of opening and closing the claws 23 of the clip 19 retained in the second region 34, namely in the horizontal direction in Fig. 5B. A surface of the arms 25 of the clip 19 in the second region 34 contacts an inner surface of the inner grooves 43a. An opening width of the inner grooves 43a is slightly greater than a maximum width of the arms 25 of the clip 19. A distance from the surface of a first one of the inner grooves 43a to that of a second one of those is substantially equal to a sum of lengths of the claws 23a of the clip 19 in the opening direction. Also, the opening width of the inner grooves 43a is smaller than a width of a wide area defined by combination of the side projections 27 on the arms 25. Therefore, the side projections 27 of the clip 19 retained in the second region 34 cannot enter the inner grooves 43a.

A distance between surfaces of the inner grooves 43a can be determined so as to maintain the engagement of the turn 26 of the first of the clips 19 with the claws 23 of the second of the clips 19, and can be smaller than a sum of a length of the claws 23 and a length of a width of a portion of the turn 26 engaged with the claws 23. The claws 23 of the clips 19 retained in the second region 34 may be overlapped on one another, and also may be kept fastened to the first of the clips 19 with a small clearance between those.

The engaged portions of two of the clips 19 are retained in a portion of the second region 34 near to its borderline on the first region 32. Among the clips 19, the clip 19B at the retaining ring 20B of Fig. 3B has the turn 26 retained by the fins 38 in the stowed position in the first region 32, and is kept from moving back and forth and from rotating. Among the clips 19, the clip 19C at the retaining ring 20B of Fig. 3B, for engagement with the clip 19B, is prevented from rotating because retained by the inner grooves 43a of the second region 34 in a direction with a difference of 90 degrees. Also, the clip 19C does not move back and forth because engaged with the clip 19B. Accordingly, the engaged portions of the two of the clips 19 are kept by the retaining ring 20 in a state with very small play.

In Figs. 5A, 5B and 5D, two end channels 44 are formed in the second region 34 at its end. The end channels 44 are disposed with a difference from the fins 38 with an angle of 90 degrees, and extend less deeply than the position of the upper end of the second region 34. In short, the end channels 44 are disposed with a difference of 90 degrees from the opening direction of the clip 19 supported with the second region 34.

Forming the end channels 44 is effective in raising the flexibility of the retaining ring 20. The flexible sheath 11 can pass a curved lumen with a small curvature in a state loaded with the multiple clip device 13. Also, fastening of the clips 19 can be easy to obtain high efficiency, because ends of the retaining ring 20 can be turned easily with the end channels 44.

The end channels 44 have a depth short of the ends of the fins 38, to prevent remarkable drop in the strength of the retaining ring 20. The end channels 44 are formed to reach a position less deep than an engaging position of the clip 19, namely an end of the clip 19 on the proximal side supported on the first region 32. It is possible to hold the clip 19 in the second region 34 of the retaining ring 20 before loading the multiple clip device 13 in the flexible sheath 11.

In Figs. 2A and 2B, the claws 23 of the clip 19B are engaged with the turn 26 of the clip 19A. Its engaged portion is retained by the retaining ring 20A. An inner surface of the retaining ring 20A in the second region 34 keeps the claws 23 closed in the clip 19B. This maintains the fastened state between the clips 19A and 19B. Similarly, the retaining ring 20B maintains the fastened state between the clips 19B and 19C. The fastened state between the clip 19C and the fastening clip mechanism 18 is maintained by the retaining ring 20C.

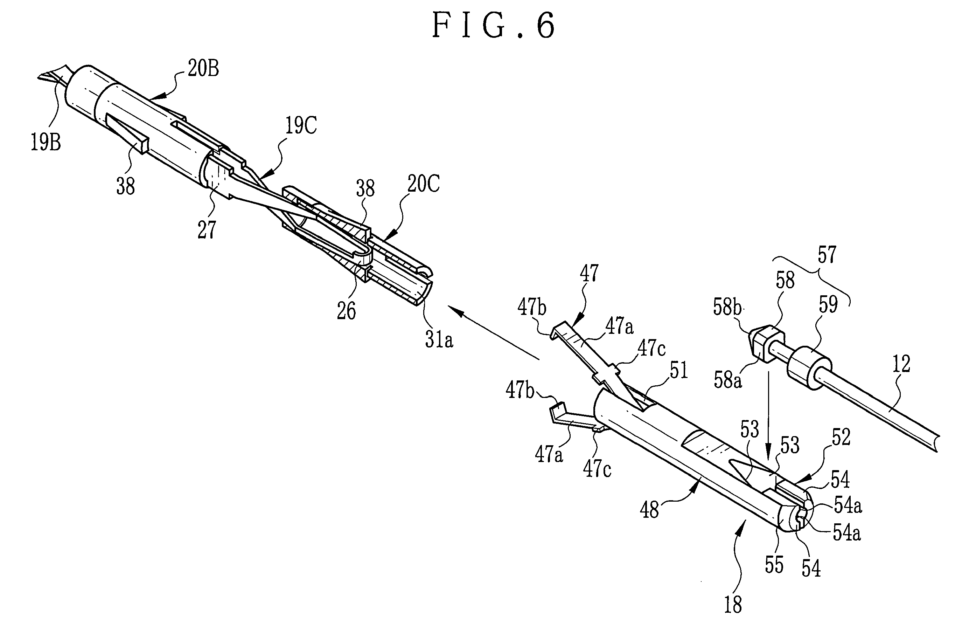

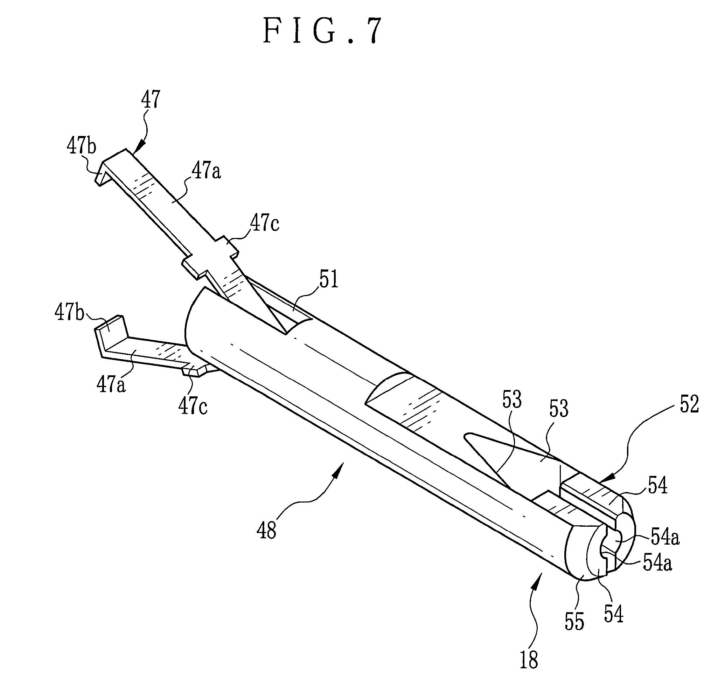

In Figs. 6 and 7, the fastening clip mechanism 18 includes a dummy clip 47 or additional clip, and a support 48 for fastening. The dummy clip 47 is engaged with the turn 26 of the clip 19C. The support 48 supports the dummy clip 47.

The dummy clip 47 is formed by bending a thin strip of a metal plate similar to the clip 19. The dummy clip 47 includes a pair of arms 47a, claws 47b, and side projections 47c. The claws 47b are disposed at ends of the arms 47a. The side projections 47c are disposed to protrude from sides of the arms 47a. The arms 47a, while left to stand without application of external force, are open in the manner of the clip 19, but become resiliently deformed and closed when pressed externally. The claws 47b have a shape equal to that of the claws 23 of the clip 19, and are engaged with the turn 26 of the clip 19C located on the proximal side. The side projections 47c have the same function as the side projections 27 of the clip 19, and contact the rear end of the retaining ring 20C at the proximal side.

The support 48 is substantially cylindrical, and formed from a material the same as that of the end connector 31 of the retaining ring 20. A support recess 51 is formed in the support 48 at its distal end for supporting the dummy clip 47. An end connector 52 is disposed at a rear end of the support 48 for connection with the operating wire 12.

The end connector 52 includes a pair of resilient walls 53 and clamping walls 54. The resilient walls 53 are shiftable resiliently crosswise to the support 48. The clamping walls 54 are disposed at ends of respectively the resilient walls 53. A clearance between the clamping walls 54 is smaller than an outer diameter of the operating wire 12. Grooves 54a of a semi-cylindrical shape are formed in respectively the clamping walls 54, and have a diameter equal to an outer diameter of the operating wire 12 with respect to the axis of the support 48.

An inclined surface 55 is disposed on an end of the clamping walls 54 and extends from a peripheral point to a proximal end to decrease the width of the clamping walls 54 gradually in the direction of insertion into the flexible sheath 11. The support 48 can be inserted easily into the flexible sheath 11 because its outer diameter is locally the smallest at the proximal end.

An example of the flexible sheath 11 is a flexible coil sheath in which a wire of metal is tightly wound in a coiled form. An inner diameter of the flexible sheath 11 is so determined that the turn 26 of a first one of the clips 19 is disengaged from the claws 23 of a second one of the clips 19. The inner diameter of the flexible sheath 11 is greater than a sum of a length of the claws 23 and a width of an engaged portion of the turn 26 with the claws 23.

The operating wire 12 is a wire of metal having biocompatibility. In Fig. 6, a shaft head 57 for hooking is disposed at the wire end of the operating wire 12 for connection with the fastening clip mechanism 18. The shaft head 57 includes a front shaft head portion 58 and a rear shaft head portion 59 arranged on the operating wire 12.

The front shaft head portion 58 includes a lateral surface 58a on a quadrilateral prismatic part, and an inclined surface 58b on a quadrilateral pyramidal part. The lateral surface 58a has one side line which has a length equal to the size of the clearance between the resilient walls 53. The inclined surface 58b has a size corresponding to the clearance between the resilient walls 53. The rear shaft head portion 59 has a cylindrical shape having a diameter which is greater than the outer diameter of the front shaft head portion 58 and slightly smaller than an outer diameter of the support 48. The rear shaft head portion 59 is distant from a proximal end of the front shaft head portion 58 by a distance which is equal to the length of the clamping walls 54 in the axial direction.

The front shaft head portion 58 is set on the end connector 52 by extending in the axial direction, and pressed into the position between the resilient walls 53 in receiving pressure in the radial direction of the support 48. The resilient walls 53 shift resiliently to tighten the connection with the front shaft head portion 58. Also, the front shaft head portion 58 is set in contact with the clamping walls 54 behind the resilient walls 53. A portion of the operating wire 12 between the front and rear shaft head portions 58 and 59 is clamped by the clamping walls 54. The rear shaft head portion 59 contacts the rear end of the clamping walls 54. Thus, pull and rotation of the operating wire 12 are transmitted to the multiple clip device 13. The multiple clip device 13 can be maintained without movement with the flexible sheath 11 by friction caused in the pull of the flexible sheath 11.

After all the clip mechanisms 17 of the multiple clip device 13 are used, the fastening clip mechanism 18 remains at the wire end of the operating wire 12. Thus, the fastening clip mechanism 18 is slid through the sheath end of the flexible sheath 11 and removed from the operating wire 12. To this end, the shaft head 57 is pulled in a radial direction away from the support 48. The front shaft head portion 58 is separated from between the resilient walls 53 to disengage the shaft head 57 from the support 48.

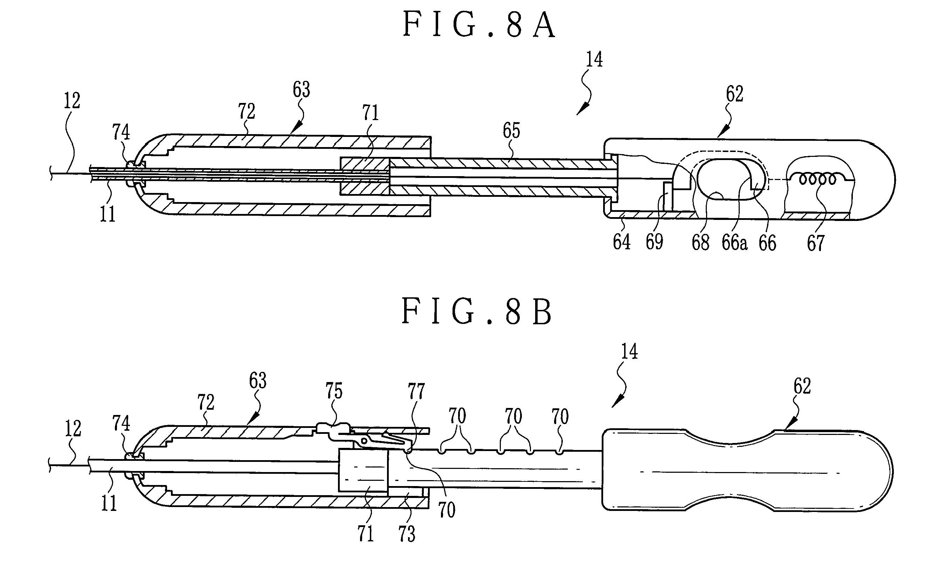

In Figs. 1, 8A and 8B, the handle assembly 14 includes a wire handle 62 and a sheath handle 63. The wire handle 62 is operable to pull the operating wire 12. The sheath handle 63 is operable to pull the flexible sheath 11. The wire handle 62 includes a handle housing 64, an elongated pipe 65, a pull arm 66 or lever, and a spring 67. The elongated pipe 65 is fixedly secured to the distal end of the handle housing 64 in a coaxial manner. The pull arm 66 and the spring 67 are contained in the handle housing 64.

The pull arm 66 is kept movable back and forth in the handle housing 64, namely in an axial direction of the wire handle 62. A window 68 is formed in the middle of the handle housing 64, through which a rear portion of the pull arm 66 appears partially. An operator inserts his or her finger to pull the pull arm 66. The spring 67 is secured to the rear end of the pull arm 66. The spring 67 is a compression coil spring, which compresses when the pull arm 66 is pulled back, but pushes the pull arm 66 forwards when the pull arm 66 is released from the finger. Thus, the pull arm 66 returns to the home position.

The window 68 defines a rear limit of movement of the pull arm 66. A pull arm surface 66a or lever surface of the pull arm 66 for touch of a finger is settable in a manner flush with the rear end of the window 68, as a position of the rear limit. Note that a stopper plate may be disposed behind the pull arm 66, for receiving the rear end of the pull arm 66 upon backward movement, to define the rear limit of its movement.

A stopper plate 69 is disposed in front of the pull arm 66 and determines a home position of the pull arm 66. The pull arm 66 is biased by the spring 67 to shift forwards, and then stops upon contacting the stopper plate 69 and returns to the home position. In Fig. 8A, the spring 67 is the compression coil spring. However, other elements can be used as the spring 67 biasing the pull arm 66 forwards, for example, leaf spring or the like.

The operating wire 12 is fixedly secured to an end of the pull arm 66 for pulling the clips 19. The operating wire 12 extends inside the sheath handle 63 and the elongated pipe 65 to the pull arm 66.

When an operator inserts a finger in the window 68 to pull the pull arm 66, the operating wire 12 is moved at the end of the pull arm 66 by its movement to the rear. The wire end of the operating wire 12 moves back. When the pull arm 66 returns to its home position by its release without pulling force, then the operating wire 12 moves similarly. The wire end comes back to the initial position.

Note that a length of pulling the operating wire 12 in the hemostatic clamping is as small as 3.1 mm. It is possible to dispose a shifting mechanism for enlarging a shifting amount of the pull arm 66 over a pulling amount of the operating wire 12, which is for the purpose of reliability in manual touch with the handle assembly 14 to an operator.

The elongated pipe 65 is a hollow part through which the operating wire 12 is inserted. A bore of the elongated pipe 65 is greater than an outer diameter of the flexible sheath 11, so as to receive insertion of the flexible sheath 11. In Fig. 8B, a plurality of notches 70 are formed in an upper side of the elongated pipe 65 and arranged at a regular interval. A distal end of the elongated pipe 65 is inserted in the sheath handle 63. A stopper ring 71 to prevent drop is fitted at the distal end of the elongated pipe 65.

In Fig. 8A, a center hole is formed in the stopper ring 71 and has a bore slightly greater than an outer diameter of the flexible sheath 11. The stopper ring 71 keeps the flexible sheath 11 movable in the axial direction.

The sheath handle 63 includes a handle housing 72, a support block 73 and a sealing ring 74. The support block 73 is disposed at a proximal end in the sheath handle 63, and supports the elongated pipe 65 inserted in the sheath handle 63. In Fig. 8B, an end face of the support block 73 contacts the stopper ring 71 secured to the elongated pipe 65, and keeps the elongated pipe 65 from moving away from the sheath handle 63.

The sealing ring 74 is disposed at an end of the handle housing 72 and on the axis of the sheath handle 63, and fixedly retains the outside of the flexible sheath 11 inserted in the sheath handle 63. When the sheath handle 63 is moved, the flexible sheath 11 moves together.

Also, the sheath handle 63 includes a button 75 and an engaging claw 77. The button 75 projects externally from the handle housing 72. The engaging claw 77 is disposed in the handle housing 72 and movable in response to movement of the button 75. The engaging claw 77 is biased toward a surface of the elongated pipe 65. One of the notches 70 in the elongated pipe 65 is engaged with the engaging claw 77, which positions the sheath handle 63 relative to the wire handle 62 in a retained manner.

When the button 75 is depressed, the engaging claw 77 is raised away from the notches 70. The sheath handle 63 becomes movable relative to the wire handle 62. Then the button 75 is released from manual touch. The sheath handle 63 is moved relative to the wire handle 62 before the engaging claw 77 is engaged with a second one of the notches 70 to disable the sheath handle 63 from moving. Thus, the sheath handle 63 on the flexible sheath 11 is adjustable stepwise with steps each of which corresponds to the interval between the notches 70. In Fig. 8, the six notches 70 are formed in the elongated pipe 65 in the handle assembly 14, and suitable for a five-clip type of multiple hemostatic clip application apparatus.

When the flexible sheath 11 is moved according to movement of the sheath handle 63, a proximal sheath end of the flexible sheath 11 on the proximal side enters the elongated pipe 65 through a center hole in the stopper ring 71. A length of the stroke of pulling the flexible sheath 11 is substantially equal to a distance between distal ends of two of the clips 19 loaded in the flexible sheath 11.

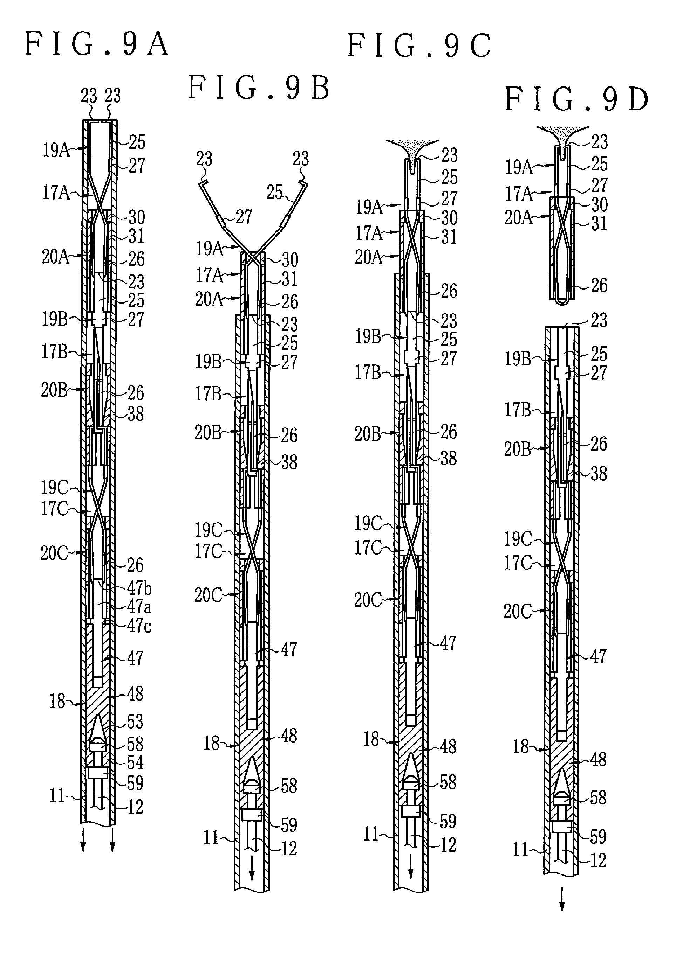

The operation of the multiple hemostatic clip application apparatus 10 is described by referring to Figs. 9A-9D. Steps of clamping of the multiple hemostatic clip application apparatus 10 are illustrated in Figs. 9A-9D one after another.

In Fig. 9A, the flexible sheath 11 is loaded with the multiple clip device 13 which includes the clip mechanisms 17A-17C and the fastening clip mechanism 18. After this, the flexible sheath 11 is inserted in a forceps channel of the endoscope. For loading of the multiple clip device 13, the support 48 of the fastening clip mechanism 18 is hooked by the shaft head 57 of the operating wire 12 protruding from the sheath end. The flexible sheath 11 is advanced relative to the operating wire 12 to contain the clip 19A fully. This loading process will be described later in detail.

In the embodiment depicted in Fig. 9A, the distal end of the clip 19A is substantially flush with the sheath end of the flexible sheath 11 in the state immediately after the loading of the multiple clip device 13. It is possible to set the clip 19A in an alternative position short of the sheath end by a predetermined length.

The clip 19A is kept closed by the inner surface of the flexible sheath 11. The arm shifting sleeve 30 of the retaining rings 20A-20C is disposed with suitable adjustment in its initial position determined near to the crossed portion 24 of the clips 19A-19C. Upper ends of the side projections 27 of the clips 19B and 19C are positioned directly under respectively the positions of the retaining rings 20A and 20C.

The sheath end of the flexible sheath 11 reaches a distal end of a head assembly of an endoscope in a human body, and comes to protrude from the head assembly. In Figs. 1, 8A and 8B, the sheath handle 63 in the handle assembly 14 is pulled to shift the engaging claw 77 from the first to the second of the notches 70. As the flexible sheath 11 is stationary on the sheath handle 63, the flexible sheath 11 moves back by a length equal to a length of movement of the sheath handle 63. Thus, the operating wire 12 does not move in contrast with the flexible sheath 11. The flexible sheath 11 can be pulled smoothly because the projections 38a operate for contact of the retaining rings 20A-20C with the flexible sheath 11.

When the flexible sheath 11 is pulled at a predetermined distance according to a distance between the first and second of the notches 70, then the sheath end comes down to a position to deploy the fins 38 of the retaining ring 20A at the distal end. The claws 23 of the clip 19A protruding from the flexible sheath 11 are deployed by resiliency to the state of Fig. 9B. Thus, the clip 19A becomes ready for use. The fins 38 of the retaining ring 20A at the distal end becomes deployed with a diameter equal to or more than a diameter of the flexible sheath 11 as illustrated in Fig. 3A.

As the engaged portions of the clips 19A and 19B are positioned directly next to the fins 38 of the retaining ring 20A in the downward direction, the distal end of the clip 19B is substantially flush with the sheath end of the flexible sheath 11 in the state of Fig. 9B.

When the flexible sheath 11 is pulled, force of friction is exerted between the flexible sheath 11 and the retaining rings 20A-20C inside. However, force of push of the clips 19 with an inner surface of the fins 38 in the stowed position is exerted to positions between the retaining rings 20A-20C and the clips 19A-19C. Also, force of push of the retaining rings 20 (the second region 34 in Figs. 5A-5D) caused by biasing of the claws 23 of the clips 19 toward the open state is exerted to the positions between the retaining rings 20A-20C and the clips 19A-19C. Also, the side projections 27 of the clips 19B and 19C and the side projections 47c of the dummy clip 47 contact proximal ends of the retaining rings 20A-20C. Thus, the retaining rings 20A-20C do not move relative to the clips 19A-19C incidentally even if the flexible sheath 11 is pulled. The retaining rings 20A-20C can maintain in the state of retaining the clips 19A-19C.

Then the multiple hemostatic clip application apparatus 10 in the state of Fig. 9B is moved. The pull arm 66 of the handle assembly 14 in Figs. 8A and 8B is pulled in a state with pressure of the claws 23 of the clip 19A in the open position to a body part to be clipped. The operating wire 12 is pulled at a predetermined length, to pull together the clip mechanisms 17A-17C engaged in the sequence from the fastening clip mechanism 18.

In the state of Figs. 9B and 9C, the fins 38 of the retaining ring 20A protruding from the flexible sheath 11 are deployed. The clip 19A is free from retention with the fins 38. The retaining ring 20A is prevented from returning into the flexible sheath 11, because the fins 38 are kept deployed by the sheath end. In Fig. 9C, the clip 19A at the distal end moves back relative to the retaining ring 20A, so that the arm shifting sleeve 30 or the end of the retaining ring 20A is positioned directly under the side projections 27 of the clip 19A. Thus, the arms 25 of the clip 19A are clamped to shift by the arm shifting sleeve 30, to clip a lesion by closing the claws 23.

At the same time as closing of the clip 19A with the retaining ring 20A is terminated, an engaged portion between the clips 19A and 19B is moved away from the end of the retaining ring 20A. Upon disengagement of the engaged portion from the retaining ring 20A, the arms 25 of the clip 19B become widely open with its resiliency to contact the inner surface of the flexible sheath 11. An interval between the claws 23 becomes greater than a width of the turn 26 of the clip 19A, to disengage the clip 19A from the clip 19B. The clip mechanism 17A including the clip 19A and the retaining ring 20A is ready to separate from the flexible sheath 11. Clamping with the clip mechanisms 17 is completed.

In the clip mechanisms 17B and 17C, the clips 19B and 19C are kept from moving both rotationally and axially with respect to the retaining rings 20B and 20C with the fins 38 in the stowed position thereon. In the clip 19C engaged with the clip 19B and in the dummy clip 47 engaged with the clip 19C, the arms 25 and the arms 47a are pressed against an inner surface of the second region 34 (Figs. 5A-5D) of the retaining rings 20B and 20C by the force of the claws 23 and the claws 47b for deployment. Also, the side projections 27 of the clip 19B and the side projections 47c of the dummy clip 47 contact the proximal ends of the retaining rings 20B and 20C. This causes high friction between the clips 19B and 19C and the retaining rings 20B and 20C. Movement of the clips 19B and 19C causes the retaining rings 20B and 20C to move together.

In short, the clip mechanisms 17B and 17C are movable back and forth together with the flexible sheath 11 in contrast with the clip mechanism 17A. The retaining rings 20B and 20C maintain the fastened state of the clips 19B and 19C and the dummy clip 47.

There is a predetermined range in which the operating wire 12 can be pulled from an initial state. The range is an amount equal to or slightly greater than a range size of the second region 34 of the retaining ring 20, and also an amount equal to or slightly smaller than a distance from a lower end of the side projections 27 of the clips 19 to an end of the retaining ring 20 holding the clips 19. The range is determined according to a distance of the home position of the pull arm 66 to a point of a rear limit of movement, in the handle assembly 14 of Fig. 8A.

The operating wire 12 is so constructed as to be pulled at a predetermined length and immediately return at the same length owing to the spring 67 biasing the pull arm 66 of the handle assembly 14. When the operating wire 12 is pulled from the state of Fig. 9B to the state of Fig. 9C, the pull arm 66 is manually released at the handle assembly 14 by terminating the pull. Then the pull arm 66 returns to its first position. The operating wire 12 returns to the state of Fig. 9D. Now the clip 19B is disposed at the distal end, and is positioned substantially at the center of the flexible sheath 11 in a manner of Fig. 9A. Similarly, the handle assembly 14 is operated in the manner of the clip mechanism 17A, to carry out the hemostatic clamping with the clip mechanisms 17B and 17C.

The dummy clip 47 is fastened to the clip 19C on the proximal side by the claws 47b which have the same form as the claws 23 of the clips 19. Disengagement of the clip 19C from the dummy clip 47 is the same as that of the clip 19A from the clip 19B and that of the clip 19B from the clip 19C. Thus, easy operability can be obtained by the present construction, as the clip mechanisms 17A-17C are commonly operable for slide in the flexible sheath 11, clamping, removal from the operating wire 12 and other steps in the operation.

As the retaining ring 20 covers the engaged portion of the clips 19, the fastened state between the clips 19 can be maintained reliably. The operating wire 12 is manually pulled in one direction for drawing the fastening clip mechanism 18 and the clip mechanisms 17 in connection, so that it is possible at the same time to close the first of the clips 19 with the arm shifting sleeve 30 of the retaining ring 20 and to disconnect the second of those. Thus, the first of the clips 19 can operate to clamp. It is possible to use a succeeding one of the clips 19 only by pulling the flexible sheath 11 to the proximal side at a predetermined length, so that next clamping is possible.

As the engaged portion of the clips 19 is covered by the retaining ring 20, inner surfaces of the flexible sheath 11 can be free from damage in contact with an angular edge of the engaged portion of the clips 19. Deformation, distortion or the like of the clips 19 can be prevented effectively at the engaged portion in the insertion of the flexible sheath 11 in an endoscope.

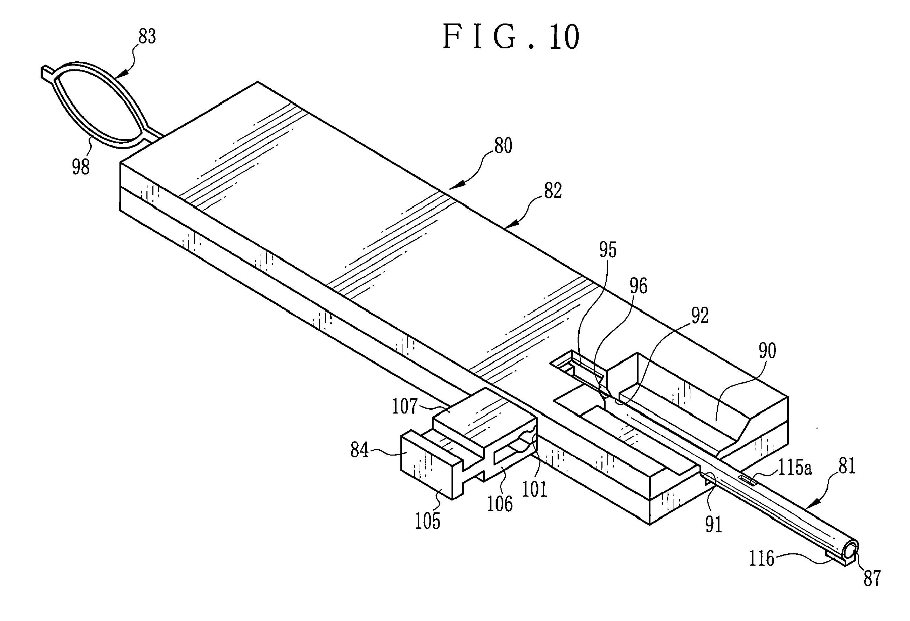

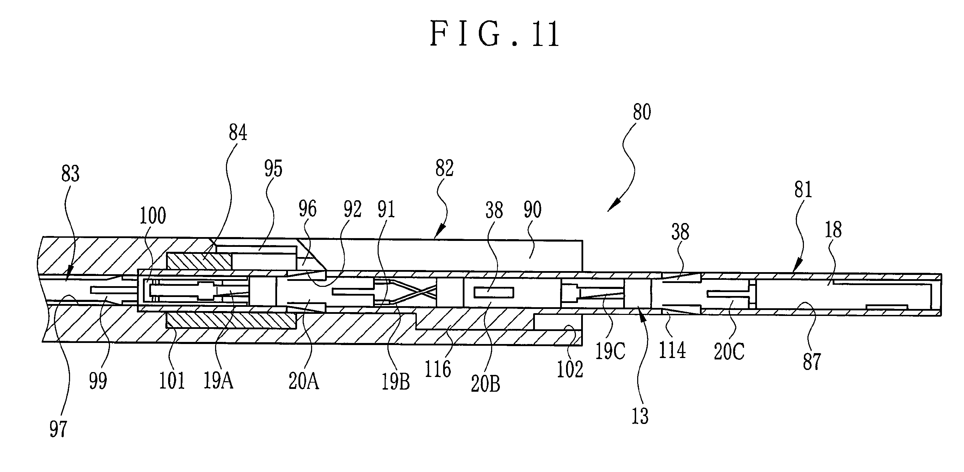

A multiple clip holder 80 or assembled clip holder or clip package for loading the flexible sheath 11 with the multiple clip device 13 is described next. In Figs. 10-12, the multiple clip holder 80 includes a housing 81 or barrel and a coupling device 82. The housing 81 contains the multiple clip device 13. The coupling device 82 has a box shape, and receives insertion of an end of the housing 81. The coupling device 82 includes a pull rod structure 83 and a slider 84. The pull rod structure 83 enters the multiple clip device 13 into the coupling device 82 from the housing 81. The slider 84 is operable for fastening the operating wire 12 to the fastening clip mechanism 18.

A through bore 87 is defined in the housing 81. An outer diameter of the housing 81 is substantially equal to an outer diameter of the flexible sheath 11. A diameter of the through bore 87 is substantially equal to the inner diameter of the flexible sheath 11. The multiple clip device 13 is contained in the through bore 87. The housing 81 also operates as a clip holder for holding the multiple clip device 13. The clip 19A in the multiple clip device 13 is disposed on the distal side of the housing 81 inserted in the coupling device 82.

The coupling device 82 operates as a body of the multiple clip holder 80, depresses the fins 38 of the multiple clip device 13 entered from the housing 81, and loads the flexible sheath 11 with the multiple clip device 13. A recess 90 is formed in the coupling device 82, and disposed at its end as viewed in the longitudinal direction, with a depth in the vertical direction. A receiving groove 91 is also formed with the recess 90 in the coupling device 82, has an inner diameter slightly greater than an outer diameter of the housing 81, and has an open upper side. An access hole 92 is formed in a wall of the recess 90 and communicates with the receiving groove 91.

The housing 81 is inserted in the receiving groove 91 and the access hole 92. For the purpose of loading the flexible sheath 11 with the multiple clip device 13 from the coupling device 82, the housing 81 is removed from the receiving groove 91 and the access hole 92 before inserting the flexible sheath 11 instead. The flexible sheath 11 or the housing 81 inserted in the receiving groove 91 appears partially at the upper portion. It is possible to retain the housing 81 and the coupling device 82 or the flexible sheath 11 and the coupling device 82 together by holding in the recess 90.

A connection opening 95 and a wire channel 96 are formed in the upper wall of the coupling device 82 in connection with the recess 90. The connection opening 95 is open for insertion of the shaft head 57 of the operating wire 12 into the coupling device 82 in a direction transverse to a direction in which the housing 81 is inserted in the coupling device 82. An open area of the connection opening 95 is larger than an area of the shaft head 57 as viewed laterally. The wire channel 96 extends for connecting the access hole 92 with the connection opening 95, and has such a dimension as to receive insertion of the operating wire 12 laterally. When the flexible sheath 11 is inserted in the receiving groove 91, the operating wire 12 and the shaft head 57 are inserted in the wire channel 96 and the connection opening 95 simultaneously, and become connected with the support 48 of the fastening clip mechanism 18 entered in the coupling device 82. See Figs. 5A-5D.

A fin bending channel 97 or skirt bending channel is formed in the coupling device 82, is positioned inwards from the access hole 92, and is a cylindrical cavity. The fin bending channel 97 has a diameter equal to an inner diameter of the flexible sheath 11 and that of the through bore 87. An inner surface of the fin bending channel 97 is set to communicate with that of the flexible sheath 11 and the housing 81 inserted in the access hole 92. The fin bending channel 97 internally depresses and stows the fins 38 of the multiple clip device 13 introduced through the through bore 87.

The pull rod structure 83 guides the multiple clip device 13 including the clips 19 from the housing 81 into the coupling device 82. The pull rod structure 83 includes a pull tab 98, a shank 99 and an end connector 100. The pull tab 98 protrudes from the coupling device 82 in its longitudinal direction, and has an elliptic shape. The shank 99 extends from the pull tab 98. The end connector 100 is formed with an end of the shank 99. The shank 99 has a length enough to penetrate the fin bending channel 97, and is inserted in the same. The end connector 100 is inserted in the through bore 87, and engaged with the clip 19A. The pull rod structure 83 is a single piece molded from a flexible plastic material.

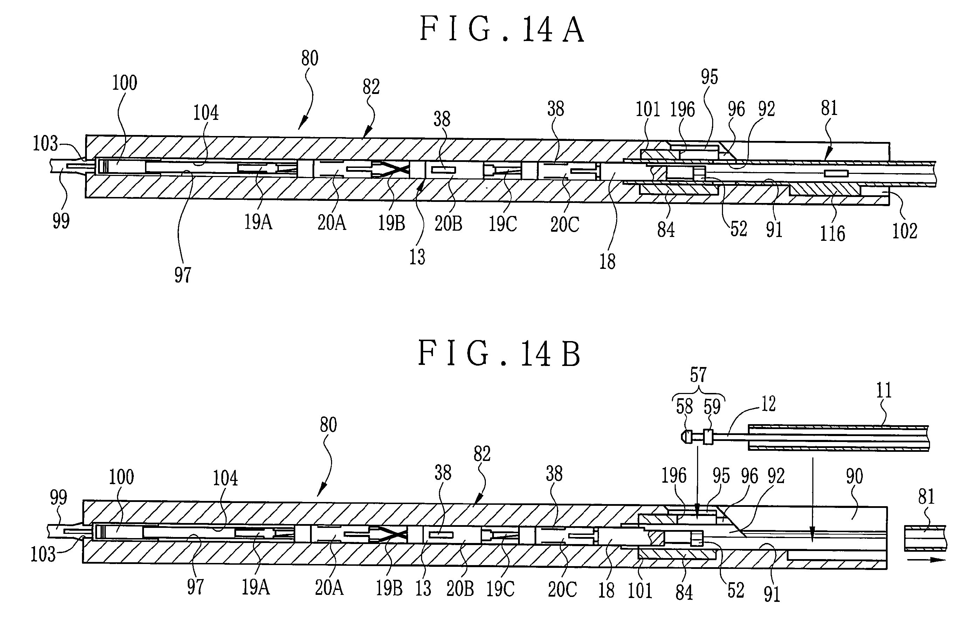

In the pull rod structure 83, the pull tab 98 is resiliently deformed in a direction of collapsing the ring shape, for insertion through the access hole 92 together with the housing 81. The shank 99 is inserted in the fin bending channel 97. A pulling hole 103 of Figs. 14A-16B is formed at the end of the fin bending channel 97. The pull tab 98 protrudes from the coupling device 82 through the pulling hole 103. When the pull tab 98 is pulled relative to the coupling device 82, the clip 19A is pulled by the end connector 100 in the multiple clip device 13 and introduced in the fin bending channel 97 through the through bore 87.

A slide channel 101 is formed through a lateral wall of the coupling device 82, and receives insertion of the slider 84 in a slidable manner. The slide channel 101 extends perpendicularly to the receiving groove 91. A shape of openness of the slide channel 101 is quadrilateral and has an opening width substantially equal to a width of the slider 84, and extends transversely to the longitudinal direction of the coupling device 82. The slider 84 operates when pressed in the slide channel 101 for connecting the fastening clip mechanism 18 entered in the coupling device 82 with the shaft head 57 inserted through the connection opening 95.

The coupling device 82 is formed from a transparent plastic material for an operator externally to view the multiple clip device 13, the housing 81, the pull rod structure 83 and the slider 84.

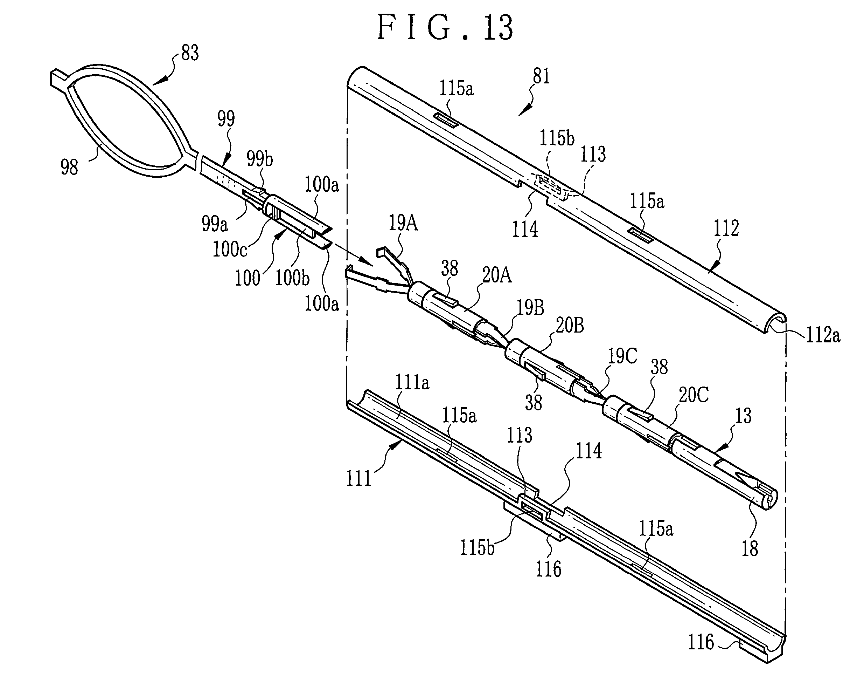

In Fig. 13, the housing 81 is constituted by a lower housing half 111 and an upper housing half 112 or barrel halves. An inner surface 111a of the lower housing half 111 and an inner surface 112a of the upper housing half 112 are semi-cylindrical, and combined to form the through bore 87 of the cylindrical shape. The through bore 87 is open at both of two ends, which include a first end for containing the clip 19A and a second end for containing the fastening clip mechanism 18.

The housing 81 is formed from a transparent plastic material for an operator externally to view the multiple clip device 13 inside the through bore 87. To join the upper housing half 112 with the lower housing half 111, it is possible to use adhesive agent for adhesion, ultrasonic waves for welding, claws for engagement and the like. Also, transparent plastic film can be wound on the periphery of the barrel obtained by combining the housing halves 111 and 112.

The multiple clip device 13 is contained in the through bore 87 while the claws 23 of the clip 19A are positioned laterally in the horizontal direction. The clip 19A is set in the closed position by the inside of the through bore 87. As has been described heretofore, the clip mechanisms 17A-17C are oriented with differences in the opening direction of the claws 23 with 90 degrees from one another. There is a difference between the claws 23 and the fins 38 in the opening direction with 90 degrees. Thus, the fins 38 of the retaining rings 20A and 20C are movable for deployment vertically in the depicted state. The fins 38 of the retaining ring 20B are movable for deployment horizontally in the depicted state.

Fin receiving slots 115a and 115b or skirt receiving slots are formed in the housing 81 for receiving the fins 38 of the retaining rings 20A-20C in a deployed position. The fin receiving slots 115a are formed in upper and lower portions of the housing 81 and receive the fins 38 of the retaining rings 20A and 20C. The fin receiving slots 115b are formed in lateral portions of the housing 81 and receive the fins 38 of the retaining ring 20B. This is effective in setting the fins 38 free from the pressing force toward the stowed position inside the housing 81 in the course of preservation. The force of recovery of the fins 38 toward the deployed position can be kept without lowering. Note that recesses or grooves may be formed in place of the fin receiving slots 115a and 115b.

The fin receiving slots 115a include two formed in the lower housing half 111 and two formed in the upper housing half 112. When the upper housing half 112 is fitted on the lower housing half 111, the fin receiving slots 115a become opposed to one another in a vertical direction. The fin receiving slots 115b include one formed in the lower housing half 111 and one formed in the upper housing half 112. When the upper housing half 112 is fitted on the lower housing half 111, the fin receiving slots 115b become opposed to one another horizontally. A ridge 113 is formed with each of the housing halves 111 and 112. Each of the fin receiving slots 115b is formed in the ridge 113. A cutout 114 is formed in each of the housing halves 111 and 112. The ridge 113 is fitted in the cutout 114 for engagement.

A positioning groove 102 is formed in a lower portion of the receiving groove 91 and extends in an axial direction. A key projection 116 under the housing 81 is inserted in the positioning groove 102 upon insertion of the housing 81 through the receiving groove 91 and the access hole 92 in the axial direction. The key projection 116 and the positioning groove 102 determine the orientation of the insertion of the housing 81 with respect to the coupling device 82 to set the multiple clip device 13 with the coupling device 82 in a predetermined rotational position.

The key projection 116 is disposed at each of the middle and end of the housing 81 as viewed in the axial direction. The key projection 116 at the middle comes to an end point of the positioning groove 102 when the end of the housing 81 comes to the innermost position of the coupling device 82, so as to position the housing 81 relative to the coupling device 82 in the axial direction.

The shank 99 of the pull rod structure 83 is inserted through the pulling hole 103 which is positioned opposite to the receiving groove 91. See Figs. 14A-16B. In Fig. 13, an end channel 99a is formed in the shank 99 of the end connector 100 and disposed near to its end. An anti-reverse projection 99b is formed to project from each of positions higher and lower than the end channel 99a, and extends with an increasing width from the pull tab 98 toward the end connector 100. The anti-reverse projection 99b extends to the outside of the coupling device 82 through the pulling hole 103 by deformation of the shank 99 at the end channel 99a with resiliency. The anti-reverse projection 99b retains the shank 99 to prevent its return into the fin bending channel 97.

The end connector 100 has an outer diameter equal to that of the retaining ring 20. A pair of guide projections 100a are included in the end connector 100 as a single piece. A wall 100b in the end connector 100 is disposed between the guide projections 100a for keeping an interval between those. Engageable projections 100c project from lateral surfaces of the wall 100b, and are disposed near to the shank 99.

The end connector 100 is contained in the through bore 87 in the housing 81 with the multiple clip device 13. The clip 19A is kept closed by pressure of the through bore 87. The claws 23 are engaged with the engageable projections 100c. When the pull tab 98 is pulled relative to the coupling device 82, the shank 99 slides in the fin bending channel 97 to move the end connector 100 from the through bore 87 into the fin bending channel 97.

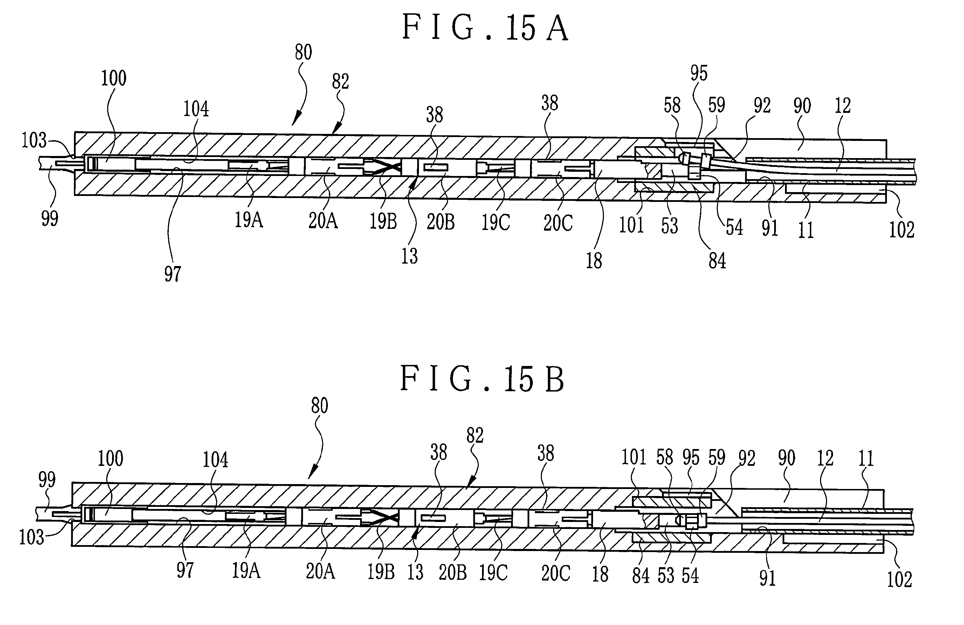

The multiple clip device 13 is pulled by the end connector 100 and introduced in the fin bending channel 97. A release groove 104 is formed in the fin bending channel 97 near to the pulling hole 103 and extends horizontally. See Figs. 14A, 14B, 15A and 15B. The release groove 104, as viewed horizontally, has such a size that the arms 25 of the clip 19A can open to disengage from the end connector 100 of the pull rod structure 83. The release groove 104 is so positioned that when the end connector 52 of the fastening clip mechanism 18 is slid to the position of the connection opening 95, the clip 19A reaches the release groove 104.

The clip 19A, upon the reach to the release groove 104, comes to shift to the open position by its resiliency, and is disengaged from the end connector 100. The multiple clip device 13 is set in the fin bending channel 97 to position the end connector 52 of the fastening clip mechanism 18 in the slide channel 101 inside the access hole 92.

Note that the clip mechanisms 17 have a difference of 90 degrees in the orientation of the claws 23 and the fins 38 for deployment and stowage. The fins 38 of the retaining ring 20A will not be deployed even while the clip 19A is disposed in the release groove 104.

The pull rod structure 83 is a single piece formed from plastic material with sufficient resiliency, inclusive of the pull tab 98, the shank 99 and the end connector 100. Note that the end connector 100 may be a component initially separate from the shank 99, and can be connected with this inside the coupling device 82.

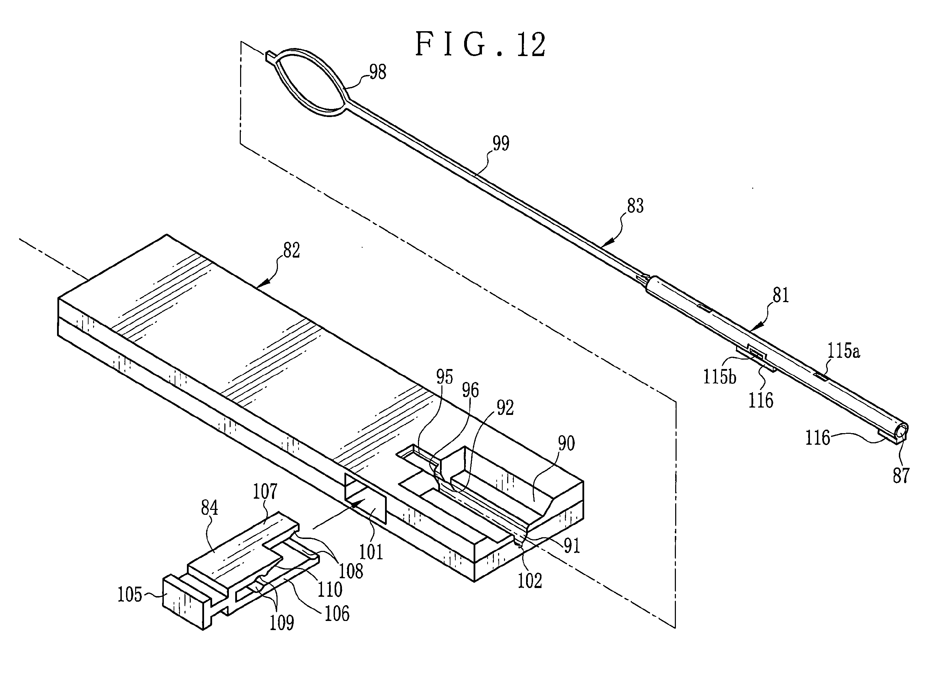

In Fig. 12, the slider 84 is a single piece molded from a plastic material, and includes a button head 105, a lower slide plate 106 and an upper slide plate 107. The button head 105 is operable for depression into the slide channel 101. The slide plates 106 and 107 are opposed to one another, and extend from the button head 105 horizontally. The slide plates 106 and 107 are inserted in the slide channel 101, and squeeze the flexible sheath 11 or the housing 81.

First receiving recesses 108 are formed in inner surfaces of the lower and upper slide plates 106 and 107. Second receiving recesses 109 are also formed and disposed in parallel with the first receiving recesses 108. The first and second receiving recesses 108 and 109 have inner diameters substantially equal to respectively the outer diameters of the flexible sheath 11 and the housing 81. The slider 84 is kept slidable between a first position and a second position, and when in the first position, registers the first receiving recesses 108 with the access hole 92, and when in the second position, registers the second receiving recesses 109 with the access hole 92.

An inclined surface 110 is formed with the upper slide plate 107 of the slider 84, presses the shaft head 57 in the access hole 92 by sliding in the slide channel 101 from the initial position to the active position, and connects the shaft head 57 with the support 48 of the fastening clip mechanism 18.

When the shaft head 57 is inserted horizontally in the connection opening 95, corners of the lateral surface 58a of the front shaft head portion 58 are inserted between the resilient walls 53 of the support 48. See Figs. 6 and 7. Advance of the slider 84 into the slide channel 101 causes the inclined surface 110 to contact the front and rear shaft head portions 58 and 59, gradually to depress those in a downward direction. The front shaft head portion 58 rotates by guiding the lateral surface 58a in contact with the inclined surface 110, resiliently to deform the resilient walls 53 for clamping. Also, the operating wire 12 spreads the clamping walls 54 and becomes clamped between those. The rear shaft head portion 59 contacts an end surface of the clamping walls 54.

A method of loading the multiple clip device 13 in the flexible sheath 11 from the multiple clip holder 80 is described now by referring to Figs. 14A-16B.

At first, the housing 81 and the coupling device 82 are held together by the recess 90. In Fig. 10, the pull tab 98 is pulled from the coupling device 82. The end connector 100 is slid to the fin bending channel 97 by following the shank 99 pulled through the coupling device 82. The multiple clip device 13 is introduced in the fin bending channel 97 through the through bore 87 by the pull of the end connector 100.

In Fig. 14A, the multiple clip device 13 is introduced in the fin bending channel 97. The fins 38 of the retaining ring 20 are depressed and stowed by the inside of the fin bending channel 97. When the clip 19A reaches the release groove 104, the arms 25 of the clip 19A become released, to disengage the claws 23 from the end connector 100. The multiple clip device 13 is stopped in the predetermined position in the fin bending channel 97. The support 48 of the fastening clip mechanism 18 reaches the position corresponding to the connection opening 95. The pull rod structure 83 is pulled as much as the anti-reverse projection 99b comes out of the coupling device 82 to protrude.

In Fig. 14B, the housing 81 is removed from the access hole 92 in the axial direction. Then a portion of the support 48 of the fastening clip mechanism 18 appears through the access hole 92. The end connector 52 of the support 48 is opposed to the connection opening 95 through the slide channel 101.

The operating wire 12 and the shaft head 57 previously protrude from the sheath end of the flexible sheath 11 according to pull of the sheath handle 63 relative to the wire handle 62. In Fig. 15A, the flexible sheath 11, the operating wire 12 and the shaft head 57 are inserted into respectively the receiving groove 91, the wire channel 96 and the connection opening 95 in the downward direction to the coupling device 82. After their insertion, the flexible sheath 11 and the coupling device 82 are retained together by use of the recess 90. Corners of the lateral surface 58a of the front shaft head portion 58 are inserted between the resilient walls 53.

When the slider 84 is pushed into the slide channel 101, the inclined surface 110 depresses the front and rear shaft head portions 58 and 59 downwards. In Fig. 15B, the front shaft head portion 58 pushed by the slider 84 becomes inserted between the resilient walls 53. The resilient walls 53 resiliently clamp the front shaft head portion 58 for tight connection. Also, the operating wire 12 is clamped between the clamping walls 54. The rear shaft head portion 59 contacts a rear end surface of the clamping walls 54.

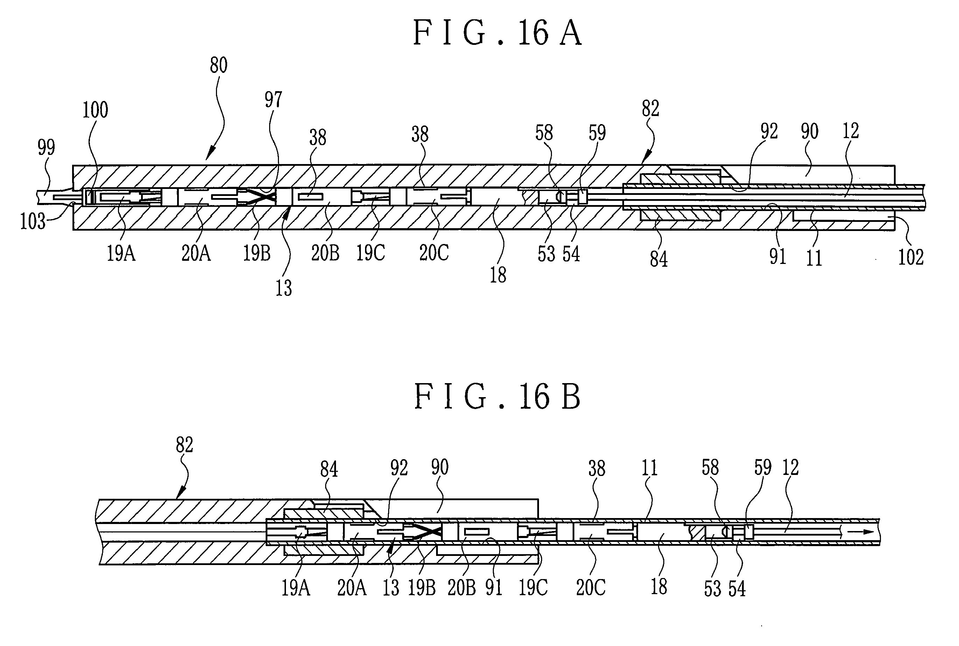

In Fig. 16A, the entirety of the multiple hemostatic clip application apparatus 10 is pushed relative to the coupling device 82. Thus, the flexible sheath 11 is pushed into the access hole 92 to reach the innermost position, for the inner surface of the flexible sheath 11 to communicate with the inner surface of the fin bending channel 97. Also, the multiple clip device 13 pushed by the rear shaft head portion 59 advances through the fin bending channel 97.

Then the operating wire 12 is pulled relative to the flexible sheath 11. For example, the wire handle 62 is pulled away from the sheath handle 63, so that the operating wire 12 can be moved relative to the flexible sheath 11 with a great length.

In Fig. 16B, the operating wire 12 is pulled. In response, the multiple clip device 13 is entered in the flexible sheath 11 by advance of its proximal end. As the inner surface of the flexible sheath 11 communicates with that of the fin bending channel 97 during loading of the multiple clip device 13, the multiple clip device 13 can be moved while the fins 38 are depressed, so that the resistance can be reduced. The multiple clip device 13 can be loaded in the flexible sheath 11 without offsetting of the retaining rings 20A-20C from the clips 19A-19C. As the proximal end of the support 48 has a decreasing diameter at the inclined surface 55, the support 48 can be entered in the flexible sheath 11 without interference with the sheath end.

When the sheath handle 63 is engaged with the first one of the notches 70 of the wire handle 62, loading of the multiple clip device 13 is completed. The flexible sheath 11 with the multiple clip device 13 is pulled away from the coupling device 82.

As has been described heretofore, a new one of the fastening clip mechanism 18 included in the multiple clip device 13 is used for fastening the operating wire 12 to the multiple clip device 13. This prevents failure in fastening in the conventional multiple hemostatic clip application apparatus due to deterioration of a retaining mechanism on the operating wire according to repeated use. Unlike the conventional multiple hemostatic clip application apparatus, it is unnecessary to exchange the relevant mechanism attached to the operating wire upon deterioration of the retaining mechanism.

Also, the support 48 is easy to insert in the flexible sheath 11 because the inclined surface 55 has the decreasing diameter on the periphery of the end connector 52. Loading of the multiple clip device 13 can be facilitated.

A second preferred embodiment of the invention is described now. Elements similar to those of the above embodiment are designated with identical reference numerals.

It is to be noted in the first preferred embodiment that the clip mechanisms 17 respectively having a length of several millimeters (mm) are moved out through the sheath end of the flexible sheath 11 one after another by pulling the flexible sheath 11 in the multiple hemostatic clip application apparatus 10. If there occurs an error in the relationship in the position between the flexible sheath 11 and the clips 19, failure in the movement of the clip mechanisms 17 is created.

The precision in positioning is high between the flexible sheath 11 and the operating wire 12, because their ends are connected with the handle assembly 14 for pull. It is necessary to raise the precision in positioning between the operating wire 12 and the multiple clip device 13 for the purpose of high precision in positioning between the flexible sheath 11 and the clip mechanisms 17.

In the multiple hemostatic clip application apparatus 10, the clips 19 are oriented with differences of 90 degrees from one another. The direction of opening and closing the claws 23 changes with 90 degrees upon advance of the clip mechanisms 17 out of the sheath end of the flexible sheath 11. It is necessary to change the opening and closing direction of the clips 19 rotationally by twisting the flexible sheath 11 and the operating wire 12 for the purpose of adapting the direction of the clips 19 to a location of a lesion. A plurality of the clip mechanisms 17 rotate together with the flexible sheath 11 by friction between the retaining rings 20 and the flexible sheath 11. If friction is different between the clip mechanisms 17, at least one of the clip mechanisms 17 may be stationary without rotation by following the flexible sheath 11. An error may occur in the fastening between the clips 19. This may cause a problem in failure in the advance of the clips 19 from the flexible sheath 11, or in damage of the clips 19.

In the embodiment, the precision in connection between the operating wire 12 and the fastening clip mechanism 18 is raised for high precision in positioning of the clip mechanisms 17 in the flexible sheath 11.

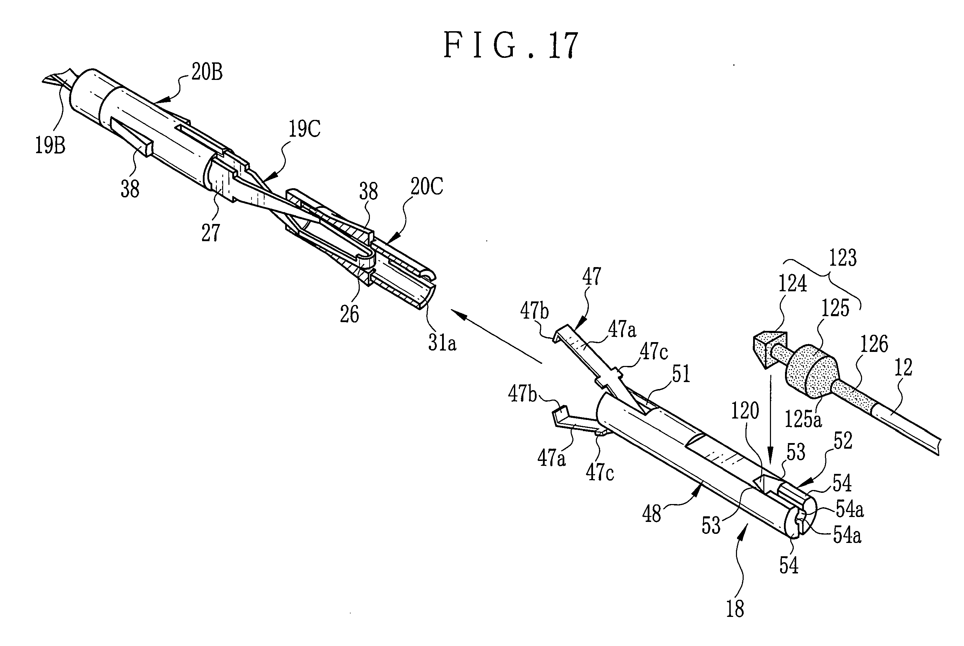

In Fig. 17, a receiving cavity 120 for securing is formed in the support 48 of the fastening clip mechanism 18. The receiving cavity 120 is in a shape of a trapezoid when viewed vertically to the axial direction of the support 48, and defined as an area surrounded by the resilient walls 53 and the clamping walls 54. The resilient walls 53 of the end connector 52 are formed to have a decreasing thickness toward the clamping walls 54 to define the receiving cavity 120.

A shaft head 123 for hooking is disposed at a wire end of the operating wire 12, and includes a front shaft head portion 124 and a rear shaft head portion 125. The shaft head 123, and the front and rear shaft head portions 124 and 125 correspond to respectively the shaft head 57, and the front and rear shaft head portions 58 and 59 of the first preferred embodiment. The front shaft head portion 124 has a shape of a frustum of a quadrangular pyramid, which is shaped in a form of a trapezoid when viewed vertically to the axial direction of the support 48 in a manner similar to the receiving cavity 120. The rear shaft head portion 125 is cylindrical and has an outer diameter which is greater than a length of a diagonal of an end of the front shaft head portion 124 on a proximal side and substantially equal to an inner diameter of the flexible sheath 11. A conical inclined surface 125a is formed with the end of the rear shaft head portion 125 on the proximal side, and has a diameter decreasing gradually toward the proximal side of the operating wire 12. There is a plating surface 126 on the shaft head 123 of the operating wire 12, for the purpose of providing high rigidity.

The front shaft head portion 124 is received in the receiving cavity 120 in the direction transverse to the axial direction of the support 48 in the same manner as the first embodiment. The operating wire 12 between the front and rear shaft head portions 124 and 125 is inserted between the clamping walls 54 by downward movement, and clamped by the inside of the grooves 54a of a semicircular shape. A distal end surface of the rear shaft head portion 125 contacts a proximal end of the clamping walls 54 when the front shaft head portion 124 and the operating wire 12 are set in respectively the receiving cavity 120 and the clamping walls 54.

When the operating wire 12 is pulled, a proximal end of the front shaft head portion 124 presses the distal ends of the clamping walls 54. The force of pulling is transmitted to the multiple clip device 13. When the operating wire 12 rotates, the front shaft head portion 124 transmits rotations to the receiving cavity 120, to rotate the multiple clip device 13 together. Also, when the flexible sheath 11 is pulled or moved back from the operating wire 12, then the distal end of the rear shaft head portion 125 retains the proximal ends of the clamping walls 54 to prevent the multiple clip device 13 from moving together with the flexible sheath 11.

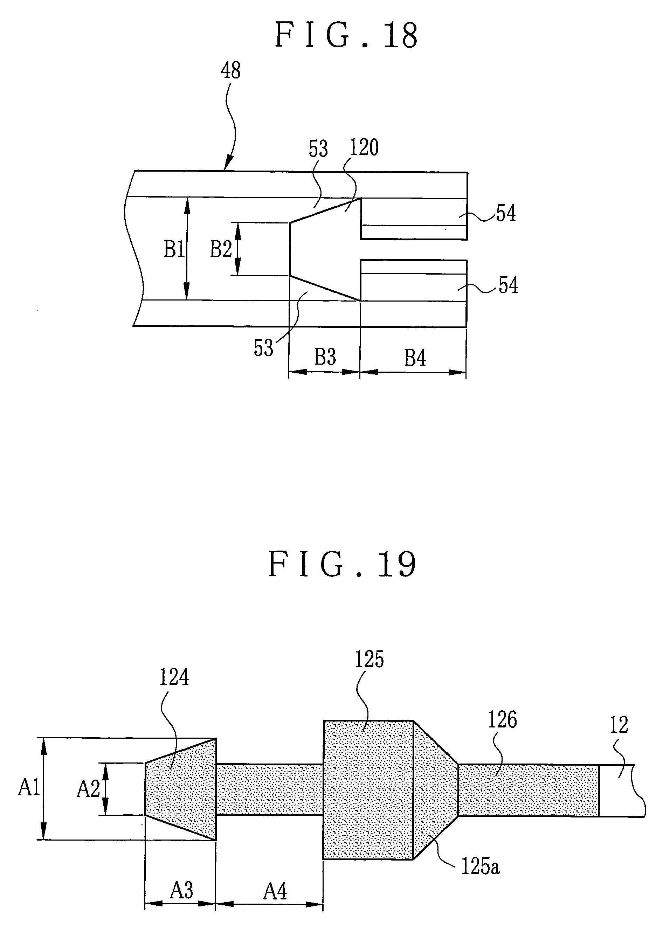

In Figs. 18 and 19, a greater width A1, a smaller width A2 and a size A3 of the front shaft head portion 124 and a greater width B1, a smaller width B2 and a size B3 of the receiving cavity 120 satisfy the conditions of A1 > B1, A2 > B2 and A3 > B3. Owing to the front shaft head portion 124 as a metal part and the receiving cavity 120 as a plastic part, the front shaft head portion 124 comes to hook the receiving cavity 120 by spreading inner surfaces of the receiving cavity 120 to be clamped. Thus, the front shaft head portion 124 is retained in the receiving cavity 120 in tight connection. Also, an interval A4 between the front and rear shaft head portions 124 and 125 and a size B4 of the clamping walls 54 satisfy the condition of A4 < B4. The interval A4 between the front and rear shaft head portions 124 and 125 slightly increases with tension applied to the wire portion between those. The clamping walls 54, when fitted on the wire portion of the interval A4, are sandwiched by the front and rear shaft head portions 124 and 125 in the axial direction. The rear shaft head portion 125 contacts the rear end of the support 48 tightly.

Thus, the shaft head 123 is connected with the support 48 without play, to raise precision in positioning the clip mechanisms 17 on the flexible sheath 11. This is effective in reducing errors in the advance of the clip mechanisms 17 by pulling the flexible sheath 11.

It is likely that the front shaft head portion 124 deforms with abrasion or the like after repeated use of the multiple hemostatic clip application apparatus 10. Considerable play may occur in connection of the front shaft head portion 124 with the support 48, seriously to lower the precision in the position of the clip mechanisms 17 relative to the flexible sheath 11.

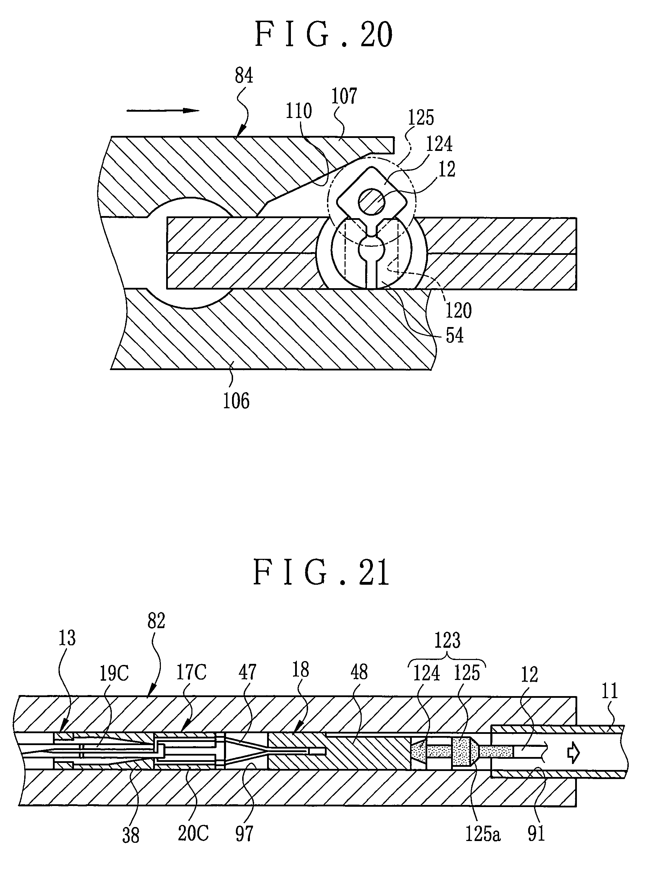

In Fig. 20, an outer diameter of the rear shaft head portion 125 is set substantially equal to an inner diameter of the flexible sheath 11. Only the rear shaft head portion 125 is caused to contact the slider 84 of the coupling device 82. The rear shaft head portion 125 is pressed against the inclined surface 110 of the slider 84. Force of pulling is transmitted to the front shaft head portion 124 by the operating wire 12, to fit the front shaft head portion 124 in the receiving cavity 120 of the support 48. Thus, the front shaft head portion 124 will not deform by abrasion or the like because of no contact with the slider 84. Note that the wire end of the operating wire 12 has high rigidity with the plating surface 126. Pressure of the rear shaft head portion 125 with the slider 84 can be transmitted to the front shaft head portion 124 reliably.

In Fig. 21, the conical inclined surface 125a guides the rear shaft head portion 125 into the flexible sheath 11 before loading the flexible sheath 11 with the multiple clip device 13. An outer diameter of the rear shaft head portion 125 can be set as great as an inner diameter of the flexible sheath 11 without lowering working efficiency in loading of the multiple clip device 13 in the flexible sheath 11.

The front shaft head portion 124 has the shape of the quadrangular pyramid. However, other forms of the front shaft head portion 124 may be used, for example a shape of a triangular or hexagonal pyramid or the like. Any suitable shape of the front shaft head portion 124 may be used for the purpose of transmitting rotations of the operating wire 12 to the support 48.

A third preferred embodiment of the invention is described now. Elements similar to those of the above embodiments are designated with identical reference numerals.

It is easy in the first preferred embodiment to carry out the fastening and disengagement, because the end connector 52 for clamping the shaft head 57 of the operating wire 12 is used to fasten the operating wire 12 to the multiple clip device 13. However, there is a problem in that the end connector 52 may drop from the operating wire 12 due to unwanted deformation in passage of the endoscope in a human body through a lumen having a very small radius of curvature. The clip mechanisms 17 will be unusable in the flexible sheath 11 upon disengagement of the operating wire 12 from the fastening clip mechanism 18.

A connector is used in the embodiment in a manner similar to the end connector 52. The connector is resiliently deformed in connection with the operating wire 12, and is characterized in easy coupling between the operating wire 12 and the fastening clip mechanism 18, and their reliably tight connection even within the flexible sheath 11.

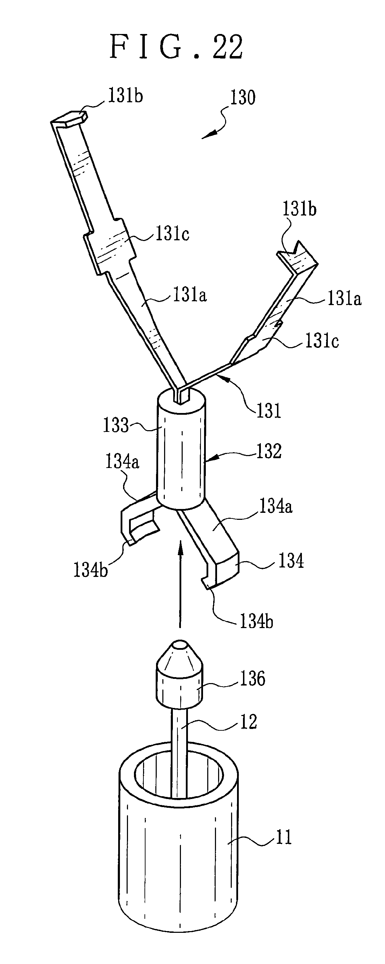

In Fig. 22, a fastening clip mechanism 130 of the preferred embodiment includes a dummy clip 131 or additional clip and a support 132 for fastening. The dummy clip 131 includes a pair of arms 131a, claws 131b and side projections 131c. The arms 131a are structurally similar to the arms 47a of the dummy clip 47, the claws 131b to the claws 47b, and the side projections 131c to the side projections 47c.

The support 132 includes a support portion 133 and an end connector 134. The support portion 133 is cylindrical and supports the dummy clip 131. The end connector 134 is open for insertion of the operating wire 12. Clamp arms 134a of the end connector 134 extend in a shiftable manner for closing. End jaws 134b are portions at ends of the clamp arms 134a for engagement with the operating wire 12. The end jaws 134b also operate for guiding the fastening clip mechanism 130 into the flexible sheath 11 so that a multiple clip device 142 or assembled clip device or clip train will be loaded in the flexible sheath 11.

At least the end connector 134 in the support 132 is formed from a deformable plastic material, for example liquid crystal polymer having rigidity and anisotropy. Thus, a coupled state of the end connector 134 with the operating wire 12 can be maintained upon closing of the end connector 134.

A shaft head 136 for hooking is disposed at the wire end of the operating wire 12, and has a decreasing diameter in the forward direction. The shaft head 136 is hooked by the end connector 134 of the support 132. The end connector 134 resiliently deforms toward its closed position for firm connection with the fastening clip mechanism 130. A material of the shaft head 136 is metal with biocompatibility, for example stainless steel.

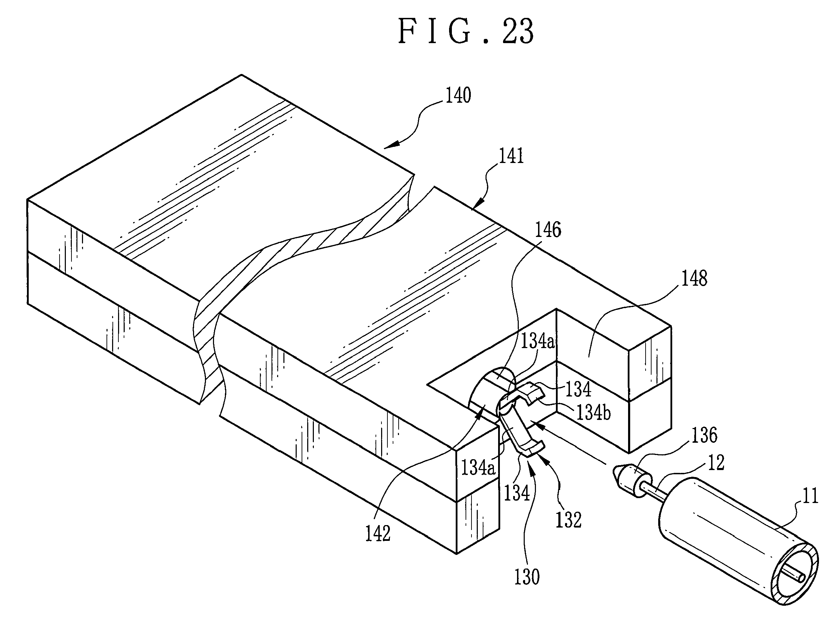

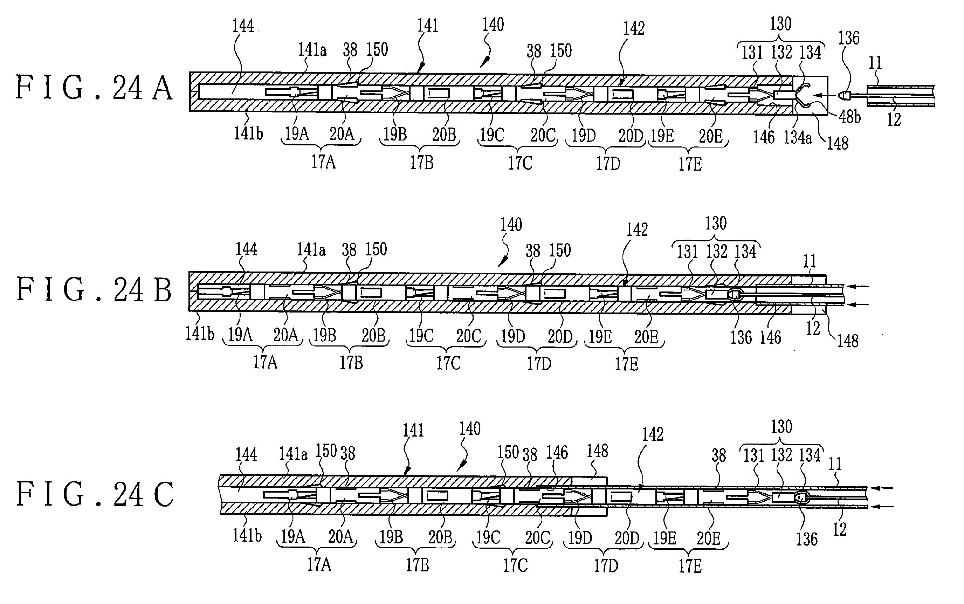

In Figs. 23 and 24A, a multiple clip holder 140 or assembled clip holder or clip package of the embodiment includes a housing 141 and the multiple clip device 142. In Figs. 23 and 24A-24C, a proximal side is located on the right side. A distal side is located on the left side. In the first embodiment, three of the clip mechanisms 17A-17C are used in the multiple hemostatic clip application apparatus 10. In the present embodiment, five including the clip mechanisms 17A-17C and hemostatic clip mechanisms 17D and 17E are used in the multiple clip device 142 in connection with the fastening clip mechanism 130.

Two housing halves 141a and 141b of a plate shape are combined to constitute the housing 141. In Fig. 24A, a through bore 144 is defined in the housing 141, and has an inner diameter substantially equal to the outer diameter of the retaining ring 20. The through bore 144 is determined by two semi-cylindrical recesses inside the housing halves 141a and 141b. The through bore 144 contains the multiple clip device 142 which includes the clip mechanisms 17A-17E and the fastening clip mechanism 130.

A receiving sleeve 146 is disposed at a proximal end of the through bore 144 and has a bore substantially equal to an outer diameter of the flexible sheath 11. Its sheath end is inserted in the receiving sleeve 146 upon loading of the multiple clip device 142 in the flexible sheath 11 through the housing 141.

A dummy clip cavity 148 or additional clip cavity is formed in a rear edge of the housing 141 and communicates with the receiving sleeve 146. The support 132 protrudes in the dummy clip cavity 148 from the fastening clip mechanism 130 contained in the receiving sleeve 146. The dummy clip cavity 148 protects the end connector 134 in the deployed position from deformation and damage even in case of incidental contact. The rear edge of the receiving sleeve 146 is open owing to the dummy clip cavity 148, to enable fastening of the operating wire 12 to the fastening clip mechanism 130 and loading of the multiple clip device 142 in the flexible sheath 11.

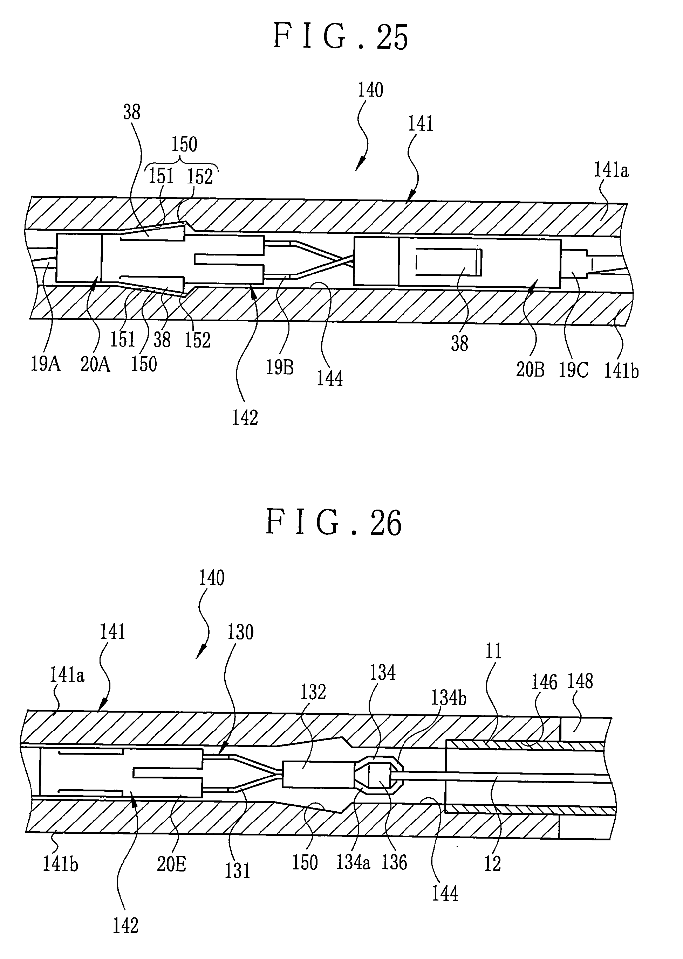

Fig. 25 is a view illustrating partial elements in Fig. 24A in enlargement. A fin receiving recess 150 or skirt receiving recess is formed in the through bore 144, is disposed in correspondence with the retaining rings 20A-20C and retaining rings 20D and 20E, and has a form suitable for the fins 38. The fin receiving recess 150 includes a first inclined surface 151 and a second inclined surface 152. The first inclined surface 151 has an inclination of an angle substantially equal to the angle of the fins 38 in a natural state, and extends radially and outwards for the inclination of the fins 38. The second inclined surface 152 extends to decrease the radius from the top end of the first inclined surface 151.

The clips 19A-19C and clips 19D and 19E are interconnected with differences of the direction at 90 degrees. In association with this, the retaining rings 20A-20E are fitted on the clips 19A-19E with differences of the direction at 90 degrees from two of the retaining rings 20 positioned at the front and back. The positions of the fin receiving recesses 150 in the housing 141 are offset with 90 degrees in the circumferential direction in positions associated with the retaining rings 20A-20E. In Fig. 24A, the fin receiving recesses 150 are illustrated in upper and lower sections corresponding to the fins 38 of the retaining rings 20A, 20C and 20E. Note that the fin receiving recesses 150 corresponding to the fins 38 of the retaining rings 20B and 20D are disposed in two sections that are arranged vertically to the drawing sheet of Fig. 24A. It is possible to form the fin receiving recesses 150 in a continuous circumferential shape positioned at the fins 38 in the horizontal direction in the drawing.

The retaining rings 20A-20E become contained in the housing 141 with the fins 38 in the deployed position in a free state because of the first inclined surface 151 of the fin receiving recesses 150. This is effective in preventing deterioration of the fins 38 with resiliency in the containment in the housing 141. The retaining rings 20A-20E can be maintained with their initial performance.

As the fin receiving recesses 150 have substantially the same shape as the fins 38, the multiple clip device 142 can be positioned inside the through bore 144 which has a greater inner diameter than an outer diameter of the retaining rings 20.

It is preferable to construct the housing 141 in a transparent or translucent form for an operator to view inner elements through its wall. The material of the housing 141 is preferably a resin free from changing in the property in a temperature range of the normal environment, for example 5-38 deg. C., in view of high resistance to shock, easy handling and high moldability. The shape of the housing 141 is not limited to the plate shape, but may be cylindrical or prismatic.

Containment of the multiple clip device 142 in the housing 141 is described now. At first, the clips 19A-19E are fastened sequentially to one another. To this end, the second region 34 of the retaining ring 20 on the clips 19 is deployed at the end channels 44. The claws 23 of the second of the clips 19 are engaged with the turn 26 of the first of the clips 19, to position their engaged portions at the retaining ring 20 suitably. In the similar manner, the fastening clip mechanism 130 is fastened to the clip 19E. Thus, the multiple clip device 142 is obtained.

The multiple clip device 142 is inserted in the housing half 141b in a state where the support 132 of the fastening clip mechanism 130 protrudes from the through bore 144 and is contained in the dummy clip cavity 148. Then the housing half 141a is fitted on the housing half 141b to obtain the housing 141.

A method of loading the multiple clip device 142 in the flexible sheath 11 from the multiple clip holder 140 is described now by referring to Figs. 24A-24C. At first, the operating wire 12 is set to protrude from the sheath end as illustrated in Fig. 24A.

It is possible to protrude the operating wire 12 from the flexible sheath 11 by operating the handle assembly 14 in Figs. 8A and 8B. In the multiple hemostatic clip application apparatus 10 of Fig. 1, after the previous hemostasis of using up all the clips 19, the handle assembly 14 of Figs. 8A and 8B is in the state of the sheath handle 63 shifted near to the wire handle 62. For example, the engaging claw 77 of the sheath handle 63 is engaged with a final one of the notches 70. A predetermined interval is defined between the wire handle 62 and the sheath handle 63. The sheath handle 63 is pulled as much as this interval to move the flexible sheath 11 relative to the operating wire 12. This causes the wire end of the operating wire 12 to protrude from the sheath end. Note that a notch may be formed in the elongated pipe 65 associated with a position of protruding the operating wire 12.

The fastening clip mechanism 130 engaged at the proximal end of the multiple clip device 142 which has been used is initially removed in a state of protruding the operating wire 12 from the flexible sheath 11.

The flexible sheath 11 and the operating wire 12 are inserted in the dummy clip cavity 148. The shaft head 136 is set within the end connector 134. Then the flexible sheath 11 is inserted in the receiving sleeve 146 while the shaft head 136 presses the support 132. The multiple clip device 142 is moved toward the distal side in the through bore 144 by pressure of the shaft head 136 to the fastening clip mechanism 130.

In Fig. 24B, the flexible sheath 11 is inserted to the position where its distal end contacts an end of the receiving sleeve 146. The end connector 134 of the support 132 resiliently deforms to close the distal end by contact of the clamp arms 134a with the through bore 144. The end jaws 134b of the end connector 134 are engaged with the proximal end of the shaft head 136. Thus, the fastening clip mechanism 130 is fastened to the operating wire 12.

When the operating wire 12 is fastened to the fastening clip mechanism 130, the flexible sheath 11 and the housing 141 in connection are moved toward the distal side relative to the operating wire 12. See Fig. 24C. An operator or person manually holds the housing 141 and advances the sheath handle 63 of the handle assembly 14 away from the wire handle 62, so as to move the flexible sheath 11 relative to the operating wire 12 toward the distal side. See Figs. 8A and 8B.

When the flexible sheath 11 and the housing 141 move toward the distal end, the operating wire 12 relatively moves toward the proximal side. In this movement, the multiple clip device 142 in connection with the operating wire 12 is drawn into the flexible sheath 11 together.

In Fig. 26, the end jaws 134b with an inclination guide the insertion of the end connector 134 into the flexible sheath 11. The fins 38 are depressed and stowed by contact of their rear edge with the second inclined surface 152. As the projections 38a of the fins 38 contact the inside of the through bore 144, their rear edge is retracted in a manner flush with or lower than an outer surface of the retaining ring 20. The multiple clip device 142 can be entered into the flexible sheath 11 without interference with the sheath end. The multiple clip device 142 can be smoothly loaded in the predetermined position because the projections 38a contact the inside of the flexible sheath 11 in the retaining ring 20 entered in the flexible sheath 11.

The second region 34 of Figs. 5A-5D is a range in the retaining rings 20A-20E disposed on the proximal side. An inner surface of the second region 34 is pressed by force of bias of the clips 19B-19E and the claws 131b of the dummy clip 131 in a deployment direction. The coupling of the clips 19 and the relationship between the clips 19A-19E and the retaining rings 20A-20E in their position are maintained in the housing 141.

As the fins 38 are stowed for loading in the flexible sheath 11, the clips 19A-19E in the retaining rings 20A-20E are pressed by inner surfaces of the fins 38. The retaining rings 20A-20E maintain the fastened state of the clips 19A-19E and the dummy clip 131. It is possible in loading in the flexible sheath 11 to prevent disengagement between the clips 19A-19E and the dummy clip 131 and prevent offsetting in the position from the retaining rings 20A-20E.

In the handle assembly 14, of Figs. 8A and 8B, the sheath handle 63 moves toward the distal side. Loading of the multiple clip device 142 is completed when the engaging claw 77 becomes engaged with a first one of the notches 70. The flexible sheath 11 with the multiple clip device 142 is pulled from the housing 141.

According to the invention, the end connector 134 is resiliently deformed at the clamp arms 134a only by insertion in the housing 141 with pressure of the shaft head 136 to the support 132. Thus, the operating wire 12 can be fastened to the fastening clip mechanism 130 easily. The end connector 134, because inserted in the upper housing half 112 by guiding of the end jaws 134b as second inclined surface, can have a reduced resistance in the insertion. The support 132, because engaged with the operating wire 12 by resilient deformation of the end connector 134, can be free from disengagement by deformation during movement in a path with a small radius of curvature.

The multiple clip holder 140 can be transported and preserved in a state connected with the clips 19, and also can be loaded in the flexible sheath 11 by simple operation in the connected state. The clips 19 can be loaded with ease, in a short time, and without very laborious operation of an operator.

As the multiple clip holder 140 can be preserved with the fins 38 of the retaining ring 20 in the deployed position, the clips 19 and the retaining ring 20 can operate with high performance for the hemostatic clamping because resiliency of the fins 38 can be maintained without deterioration. Clip loading with the multiple clip holder 140 is easy, because the fins 38 can be stowed before loading into the flexible sheath 11.

The support 132, when located outside the housing 141, can be resiliently deformed by force of fingers or the like and by insertion of the shaft head 136 in the end connector 134. In the above embodiments, the flexible sheath 11 is loaded with the multiple clip device 142 by advance relative to the operating wire 12. However, the flexible sheath 11 may not be moved for this purpose. The operating wire 12 can be pulled to enter the multiple clip device 142 in the flexible sheath 11.

The present invention is not limited to the construction in which the clips 19 are fastened with a difference of an angle of 90 degree between two of those. For example, a specific clip can be used between the claws 23 and the turn 26 and has a form twisted at 90 degrees. Two of the clips 19 next to one another may be fastened in the same orientation by use of the specific clip.

The use of the first type of the clip having the turn is preferable because pressure to the turn can exert force of bias to open the arms, the first type being referred to as a closed clip having the turn. However, it is possible in the invention for a clip to be a second type without a turn, the second type including two separate pieces combined in a U shape but without a turn, and being referred to as an open clip.

Furthermore, an endoscope for use with the multiple clip application apparatus of the invention may be a rigid endoscope instead of a flexible endoscope, the apparatus being any one of a multiple clip application apparatus including a multiple clip holder of the invention, and a multiple clip application apparatus operable according to a clip loading method of the invention.

The following is a preferable mode according to the second preferred embodiment.
1. A multiple clip application apparatus comprising:
   an operating wire, inserted in a cylindrical flexible sheath, and having a first shaft head portion with a decreasing width;
   a multiple clip device, having plural clips fastened to one another in a train, and an end connector disposed on a final one of the clips disposed finally, wherein the first shaft head portion is fitted in the end connector for connection with the operating wire and loading in the flexible sheath.

The following are preferable modes according to the third preferred embodiment.
2. A dummy clip comprising:
   a support fastened to a proximal end of a clip contained in a housing on a proximal side; and
   a connector for receiving insertion of a shaft head at a distal end of an operating wire for operating the clip, and for connection with the shaft head by resilient deformation.
3. A dummy clip as defined in embodiment mode 2, wherein the connector is pressed by the shaft head, inserted in the housing, and resiliently deformed by contacting an inner surface of the housing.
4. A dummy clip as defined in embodiment mode 3, wherein the connector includes a pair of clamp arms openable for insertion of the shaft head, and each of the clamp arms includes a first inclined surface for contacting the inner surface of the housing upon insertion therein to close the clamp arms, and an end jaw, formed with an end of the clamp arms, for engagement with the shaft head upon closing the clamp arms.
5. A dummy clip as defined in embodiment mode 4, wherein the end jaw includes a second inclined surface for guiding insertion of the clamp arms relative to a cylindrical flexible sheath after connection of the shaft head to the connector according to operation for entry of the operating wire in the flexible sheath.
6. A multiple clip holder comprising:
   a multiple clip device, having plural clips engaged with one another in a train, plural retaining rings for keeping the clips fastened by covering engaged portions of the clips, and the dummy clip defined in any one of embodiment modes 3, 4 and 5 and fastened to a final one of the clips disposed finally; and
   a housing, having a through bore, having an inner diameter slightly greater than an outer diameter of the retaining rings, for containing the multiple clip device, and a dummy clip cavity for containing the connector to receive access of the shaft head.
7. A multiple clip holder as defined in embodiment mode 6, wherein the housing includes a receiving sleeve for fastening of an end of the flexible sheath.
8. A clip loading method of loading the multiple clip device in the flexible sheath from the multiple clip holder as defined in embodiment mode 7, comprising steps of:
   fastening the shaft head portion to the dummy clip by resiliently deforming the connector upon insertion in the housing with pressure of the shaft head portion to the connector;
   loading the multiple clip device in the flexible sheath from the housing by moving the flexible sheath and the housing toward a distal end side relative to the operating wire after setting the flexible sheath in the receiving sleeve of the housing.

Although the present invention has been fully described by way of the preferred embodiments thereof with reference to the accompanying drawings, various changes and modifications will be apparent to those having skill in this field. Therefore, unless otherwise these changes and modifications depart from the scope of the present invention, they should be construed as included therein.

## Claims

1. A multiple clip device comprising:
plural clips (19) fastened to one another in a train, having first and second clips, a proximal end portion (26) of said first clip (19A) being engaged with two claws (23) of a distal end portion of said second clip (19B-19E);
a dummy clip (47, 131), having claws (47b, 131b) in a shape of said claws of said clips, and engaged with a proximal end portion of a final clip among said clips by said claws thereof; and
an end connector (52, 134) for retention of said dummy clip, and for fastening in a removable manner to a shaft head (57, 123, 136) at an end of an operating wire (12) inserted in a flexible sheath (11) of a clip application apparatus (10).

2. A multiple clip device as defined in claim 1, wherein said end connector has a proximal end portion inclined with a decreasing diameter in a direction of insertion into said flexible sheath.

3. A multiple clip device as defined in claim 1, wherein said shaft head includes a first shaft head portion with a decreasing width at a distal end portion, and said first shaft head portion is fitted in said end connector for connection with said operating wire.

4. A multiple clip device as defined in claim 3, wherein said shaft head includes a second shaft head portion, positioned behind said first shaft head portion, for contacting a proximal end of said end connector;
said end connector includes a clamping wall engaged with a proximal end of said first shaft head portion by clamping said operating wire between said first and second shaft head portions.

5. A multiple clip device as defined in claim 4, wherein an interval between said first and second shaft head portions is smaller than a length of said clamping wall in an axial direction.

6. A multiple clip application apparatus comprising:
an operating wire (12), inserted in a flexible sheath (11), and having a shaft head (57, 123, 136) at an end thereof;
a multiple clip device (13, 142) connected with said operating wire, and loaded in said flexible sheath;
said multiple clip device including:
plural clips (19) fastened to one another in a train, having first and second clips, a proximal end portion (26) of said first clip (19A) being engaged with two claws (23) of a distal end portion of said second clip (19B-19E);
a dummy clip (47, 131), having claws (47b, 131b) in a shape of said claws of said clips, and engaged with a proximal end portion of a final clip among said clips by said claws thereof; and
an end connector (52, 134) for retention of said dummy clip, and for fastening in a removable manner to said shaft head.

7. A multiple clip application apparatus as defined in claim 6, wherein said end connector has a proximal end portion inclined with a decreasing diameter in a direction of insertion into said flexible sheath.

8. A multiple clip application apparatus as defined in claim 6, wherein said shaft head includes a first shaft head portion with a decreasing width at a distal end portion, and said first shaft head portion is fitted in said end connector for connection with said operating wire.

9. A multiple clip application apparatus as defined in claim 8, wherein said shaft head includes a second shaft head portion, positioned behind said first shaft head portion, for contacting a proximal end of said end connector;
said end connector includes a clamping wall engaged with a proximal end of said first shaft head portion by clamping said operating wire between said first and second shaft head portions.

10. A multiple clip application apparatus as defined in claim 9, wherein an interval between said first and second shaft head portions is smaller than a length of said clamping wall in an axial direction.

11. A multiple clip application apparatus as defined in claim 9, wherein said flexible sheath is substantially cylindrical;
said second shaft head portion is substantially cylindrical, and has a diameter greater than a length of a diagonal of said proximal end of said first shaft head portion and substantially equal to an inner diameter of said flexible sheath, and said first shaft head portion is engaged with said end connector by pressing said second shaft head portion.

12. A multiple clip application apparatus as defined in claim 11, wherein said second shaft head portion includes an inclined surface, disposed at an end thereof on a proximal side, having a gradually decreasing diameter toward said proximal side in said operating wire.

13. A multiple clip application apparatus as defined in claim 9, further comprising plating provided on a distal end portion of said operating wire including said first and second shaft head portions.
